(19) Europäisches Patentamt
European Patent Office
Office européen des brevets

(11) Veröffentlichungsnummer: **0 287 890**
**A1**

(12) # EUROPÄISCHE PATENTANMELDUNG

(21) Anmeldenummer: **88105456.3**

(22) Anmeldetag: **06.04.88**

(51) Int. Cl.4: **C07D 207/337 , C07D 207/333 , A61K 31/40**

(30) Priorität: **14.04.87 DE 3712720**
**10.08.87 DE 3726528**
**13.11.87 IT 2263687**

(43) Veröffentlichungstag der Anmeldung:
**26.10.88 Patentblatt 88/43**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Hübsch, Walter, Dr.**
**Am Eckbusch 43/50**
**D-5600 Wuppertal 1(DE)**
Erfinder: **Angerbauer, Rolf, Dr.**
**Sterntalerweg 29**
**D-5600 Wuppertal 1(DE)**
Erfinder: **Fey, Peter, Dr.**
**Ursulastrasse 14**
**D-5600 Wuppertal 1(DE)**
Erfinder: **Bischoff, Hilmar, Dr.**
**Am Röhm 78**
**D-5600 Wuppertal 2(DE)**
Erfinder: **Petzinna, Dieter, Dr.**
**Peter-Berten-Strasse 10**
**D-4000 Düsseldorf 12(DE)**
Erfinder: **Schmidt, Delf, Dr.**
**Am Eckbusch 55b**
**D-5600 Wuppertal 1(DE)**
Erfinder: **Thomas, Günter, Dr.**
**Via Campogallo 21/14**
**I-2020 Arese (Mi)(IT)**

(54) **Substituierte Pyrrole.**

(57) Die neuen substituierten Pyrrole können durch Reduktion von Ketonen, die durch einen Pyrrolrest substituiert sind, und nachfolgender Verseifung, Cyclisierung oder Hydrierung hergestellt werden. Sie können als Wirkstoffe in Arzneimitteln verwendet werden.

EP 0 287 890 A1

Xerox Copy Centre

## Substituierte Pyrrole

Die Erfindung betrifft substituierte Pyrrole, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Es wurden nun substituierte Pyrrole der allgemeinen Formel (I),

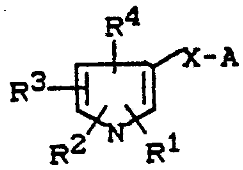

(I)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroraryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

wobei

$R^3$ - auch für einen Alkylrest stehen kann,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylami nocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy,Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy,

Alkyl, Alkoxy, Alkylthio oder Alkysulfonyl substituiert sein können,
oder
-fur Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl, oder durch eine Gruppe der Formel $-NR^5R^6$ substituiert sein kann,
worin
$R^5$ und $R^6$ die oben angegebene Bedeutung haben,
oder
-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
X - für eine Gruppe der Formel $-CH_2-CH_2-$oder $-CH=CH-$steht,
und
A - für eine Gruppe der Formel

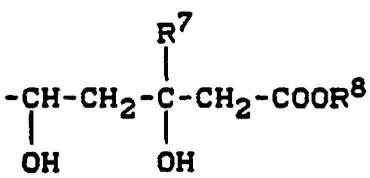

oder

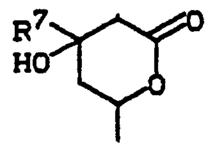

steht,
worin
$R^7$ -Wasserstoff oder Alkyl bedeutet
und
$R^8$ -Wasserstoff,
-Alkyl, Aryl oder Aralkyl oder
-ein Kation bedeutet,
gefunden.

Überraschenderweise zeigen die erfindungsgemäßen substituierten Pyrrole eine gute inhibitorische Wirkung auf die HMG-CoA Reduktase (3-Hydroxy-3-methyl-glutaryl Coenzym A Reduktase) und bewirken eine Senkung des Cholesteringehaltes im Blut.

Cycloalkyl steht im allgemeinen für einen cyclischen Kohlenwasserstoffrest mit 3 bis 8 Kohlenstoffatomen. Bevorzugt ist der Cyclopropyl-, Cyclopentan-und der Cyclohexanring. Beispielsweise seien Cyclopropyl, Cyclopentyl, Cyclohexyl, Cycloheptyl und Cyclooctyl genannt.

Alkyl steht im allgemeinen für einen verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niedrigalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Aryl steht im allgemeinen für einen aromatischen Rest mit 6 bis etwa 12 Kohlenstoffatomen. Bevorzugte

Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen, wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethlysulfonyl und Phenylpropylsulfonyl.

Alkoxycarbonyl kann beispielswiese durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niedrigalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor, oder Brom. Besonders bevorzugt steht Halogen für Fluor oder Chlor.

Heteroaryl im Rahmen der oben angebenen Definition steht im allgemeinen für einen 5-bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5-und 6-gliedrige aromatische Ringe, die einen Sauerstoff, ein Schwefel und/oder bis zu 2 Stickstoff atome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl und Isoindolyl.

$R^8$ steht für Alkyl, Aryl oder Aralkyl und bildet hiermit bevorzugt einen physiologisch verträglicher Esterrest, der in vivo leicht zu einer freien Carboxylgruppe und einem entsprechenden physiologisch verträglichen Alkohol hydrolysiert wird. Hierzu gehören beispielsweise Alkylester ($C_1$ bis $C_4$) und Aralkylester ($C_7$ bis $C_{10}$), bevorzugt Niederalkylester sowie Benzylester. Darüber hinaus seien die folgenden Esterreste genannte:

Methylester, Ethylester, Propylester, Benzylester.

Steht $R^8$ für ein Kation, so ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium-oder Calciumkationen, sowie Aluminium-oder Ammoniumkationen, sowie nicht-toxische substituierte Ammoniumkationen aus Aminen wie Diniedrigalkylamine, Triniedrigalkylamine, Prokain, Dibenzylamin, N,N′-Dibenzylethylendiamin, N-Benzyl-$\beta$-phenylethylamin, N-Methylmorpholin oder N-Ethylmorpholin,

1-Ephenamin, Dihydroabiethylamin, N,N'-Bis-dihydroabiethylethylendiamin, N-Niedrigalkylpiperidin und andere Amine, die zur Bildung von Salzen verwendet werden können.

Im Rahmen der vorliegenden Erfindung entsprechen die substituierten Pyrrole (Ia) der allgemeinen Formel

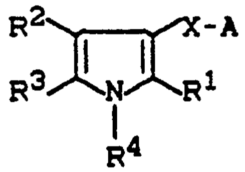

(Ia)

in welcher
$R^1$, $R^2$, $R^3$, $R^4$, X und A die oben angegebene Bedeutung haben.

Im Rahmen der vorliegenden Erfindung entsprechen die N-substituierten Pyrrole (Ib) der allgemeinen Formel

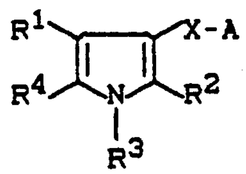

(Ib)

in welcher
$R^1$, $R^2$, $R^3$, $R^4$, X und A die oben angegebene Bedeutung haben.

Im Rahmen der allgemeinen Formel (I) sind Verbindungen mit dem allgemeinen Formeln (Ia) und (Ib) bevorzugt.

Bevorzugt sind solche Verbindungen der allgemeinen Formel (Ia) und (Ib)

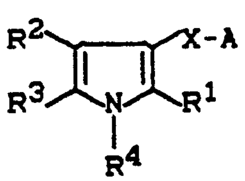

(Ia) oder

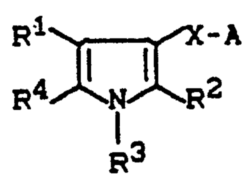

(Ib)

in welchen
$R^1$ - für Cyclopropyl, Cyclopentyl oder Cyclohexyl steht, oder

-für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ gleich oder verschieden sind und Niederalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenyl ethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,
$R^2$ und $R^3$ gleich oder verschieden sind und

-für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder

-für Phenyl oder Naphthyl stehen, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ die oben angegebene Bedeutung haben,
wobei $R^3$ auch für Niederalkyl stehen kann,
$R^4$ -für Wasserstoff oder
-für Benzoyl oder Niederalkylcarbonyl steht, oder
-für Niederalkylsulfonyl,Phenylsulfonyl oder Tolylsulfonyl steht, oder
-für Aminocarbonyl, Niederalkylaminocarbonyl, Diniederalkylaminocarbonyl, Phenylaminocarbonyl oder Niederalkoxycarbonyl steht, oder
-für Cyclopropyl, Cyclopentyl oder Cyclohexyl steht oder
-für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ gleich oder verschieden sind und Niederalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl. Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,
-für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
-für Phenyl oder Naphthyl stehen, die bis zu 4-fach gleich oder verschieden substituiert sein können durch Niederalkyl, Niederalkoxy, Nideralkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ die oben angegebene Bedeutung haben,
X - für eine Gruppe der Formel $-CH_2-CH_2-$oder $-CH=CH-$steht,
A - für eine Gruppe der Formel

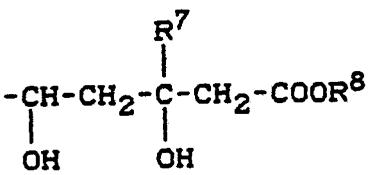

oder

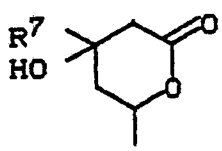

steht,
worin
$R^7$ -Wasserstoff oder Niederalkyl bedeutet,
und
$R^8$ -Wasserstoff,
-Alky, ($C_1$-$C_4$), Aryl($C_6$-$C_{12}$), Aralkyl ($C_7$-$C_10$) oder
-ein physiologisch verträgliches Kation bedeutet.
Besonders bevorzugt sind Verbindungen der allgemeinen Formeln (Ia) und (Ib) ,
in welchen
$R^1$ - für Cyclopropyl, Cyclopentyl oder Cyclohexyl steht, oder
-für Methyl, Ethyl, Propyl, Isopropyl, Butyl, sec.Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^2$ und $R^3$ gleich oder verschieden sind und

-für Pyridyl, Pyrimidyl, Chinolyl oder Isochinolyl stehen, die durch Fluor, Chlor, Methyl, Methoxy oder Trifluormethyl substituiert sein können, oder

-für Phenyl stehen, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxy carbonyl, Isobutoxycarbonyl oder tert.Butoxycarbonyl,

wobei $R^3$ auch für Methyl, Ethyl, Propyl, iso-Propyl, Butyl und iso-Butyl,

$R^4$ -für Wasserstoff, Cyclopropyl, Cyclopentyl oder Cyclohexyl steht, oder

-für Methyl, Ethyl, Propyl, Isopropyl, Butyl, Isobutyl, tert.Butyl, Pentyl, Isopentyl, Hexyl oder Isohexyl steht, die substituiert sein können durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutyoxycarbonyl, tert.Butoxycarbonyl, Benzoyl, Acetyl, Ethylcarbonyl, oderdurch eine Gruppe $-NR^5R^6$,

wobei

$R^5$ und $R^6$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl, oder Phenylsulfonyl bedeuten,

oder durch Pyridyl, Pyrimidyl, Pyrazinyl, Pyridazinyl, Chinolyl, Isochinolyl, Pyrryl, Thienyl, Furyl, Phenyl, Phenoxy, Phenylthio, Phenylsulfonyl, Benzyloxy, Benzylthio oder Benzylsulfonyl, wobei die genannten Heteroaryl-und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl, oder Trifluormethoxy substituiert sein können, oder

-für Thienyl, Furyl, Pyridyl, Pyrimidyl, Pyrazinyl, Pyridazinyl; Oxazolyl, Isooxazolyl, Imidazolyl, Pyrazolyl, Thiazolyl, Isothiazolyl, Chinolyl, Isochinolyl, Benzoxazolyl oder Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder

-für Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutyoxycarbonyl, tert.Butoxycarbonyl oder durch eine Gruppe $-NR^5R^6$ substituiert sein kann,

wobei

$R^5$ und $R^5$ die oben angegebene Bedeutung haben,

oder

-für Benzoyl, Acetyl, Ethylcarbonyl, Propylcarbonyl, Isopropylcarbonyl, Methylsulfonyl, Ethylsulfonyl, Propylsulfonyl, Isopropylsulfonyl, Butylsulfonyl, Isobutylsulfonyl, tert.Butylsulfonyl, Phenylsulfonyl oder Tolylsulfonyl steht,

-für Aminocarbonyl, Methyl-, Ethyl-, Propyl-, Isopropyl-, Butyl-, Isobutyl-oder tert.Butylaminocarbonyl, Dimethyl-, Diethyl-, Dipropyl-, Dipropyl-, Diisopropyl-, Dibutyl-oder Diisobutylaminocarbonyl, Phenylaminocarbonyl,Methoxycarbonyl, Ethoxycarbonyl, Propoxycarbonyl, Isopropoxycarbonyl, Butoxycarbonyl, Isobutoxycarbonyl oder tert.Butoxycarbonyl steht,

X - für eine Gruppe der Formel $-CH_2-CH_2-$oder $-CH=CH-$steht,

A - für eine Gruppe der Formel

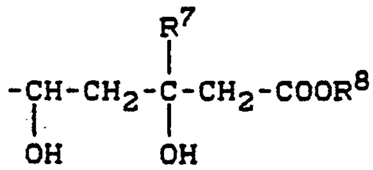 oder 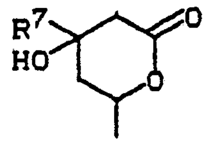

steht,
worin
$R^7$ -Wasserstoff, Methyl, Ethyl, Propyl, Isopropyl, Butyl, Isobutyl oder tert.Butyl bedeutet,
und
$R^8$ -Wasserstoff, Methyl, Ethyl, Propyl, Isopropyl, Butyl, Isobutyl oder tert.Butyl oder Benzyl bedeutet, oder ein Natrium-, Kalium-, Calcium -, oder Magnesium-oder Ammoniumion bedeutet.

Die erfindungsgemäßen substituierten Pyrrole der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Je nach der Bedeutung der Gruppe X bzw. des Restes A ergeben sich unterschiedliche Stereoisomere, die im folgenden näher erläutert werden sollten:

a) Steht die Gruppe -X-für eine Gruppe der Formel -CH=CH-, so können die erfindungsgemäßen Verbindungen in zwei stereoisomeren Formen existieren, die an der Doppelbindung E-konfiguriert (II) oder Z-konfiguriert (III) sein können:

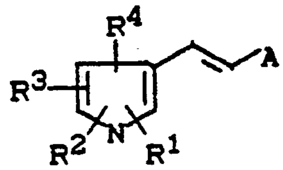 (II) E-Form

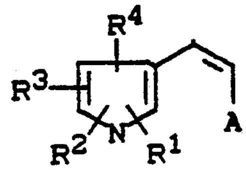 (III) Z-Form

($R^1$ bis $R^4$, A haben die oben angegebene Bedeutung).
Bevorzugt sind diejenigen Verbindungen der allgemeinen Formel (I) die E-konfiguriert sind (II).

b) Steht der Rest -A-für eine Gruppe der Formel

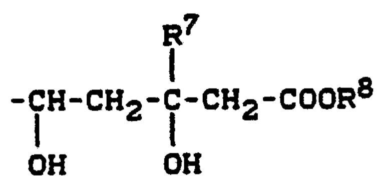

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

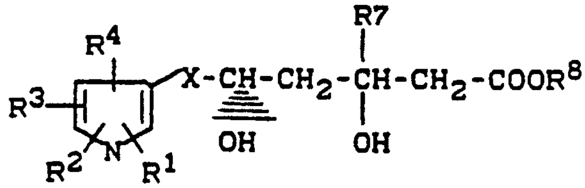

Erythro-Form (IV)

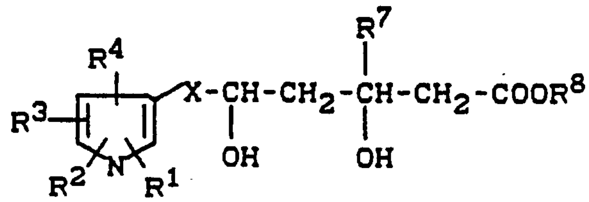

Threo-Form (V)

Sowohl von den Verbindungen in Erythro-als auch in Threo-Konfiguration existieren wiederum jeweils zwei Enantiomere, nämlich 3R,5S-Isomeres bzw. 3S,5R-Isomeres (Erythro-Form) sowie 3R,5R-Isomeres und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -A-für eine Gruppe der Formel

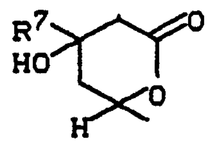

so besitzen die substituierten Pyrrole mindestens zwei asymmetrische Kohlenstoffatome, nämlich das Kohlenstoffatom an dem die Hydroxygruppe gebunden ist, und das Kohlenstoffatom an welchem der Rest der Formel

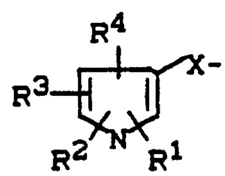

gebunden ist. Je nach der Stellung der Hydroxygruppe zur freien Valenz am Lactonring können die substituierten Pyrrole als cis-Lactone (VI) oder als trans-Lactone (VII) vorliegen.

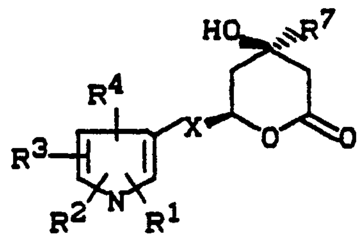

cis-Lacton (VI)

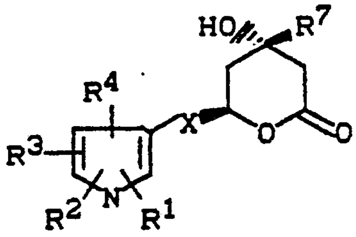

trans-Lacton (VII)

Sowohl vom cis-Lacton aus als auch vom trans-Lacton existieren wiederum jeweils zwei Isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrrole genannt:

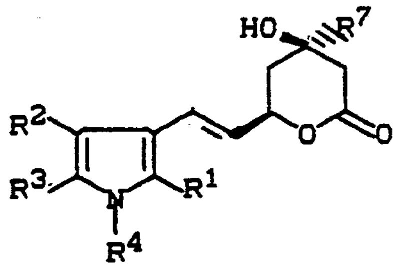

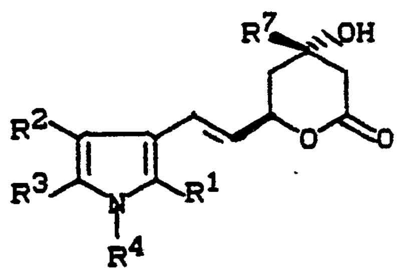

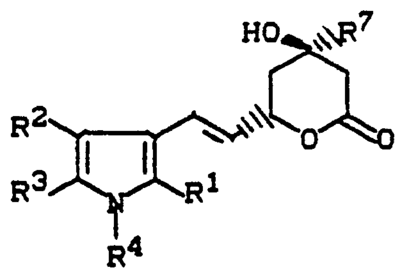

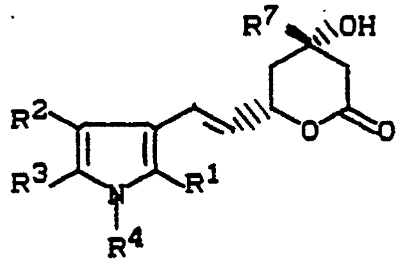

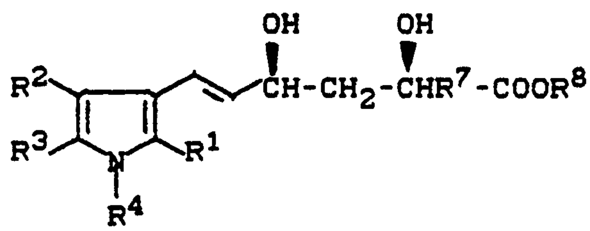

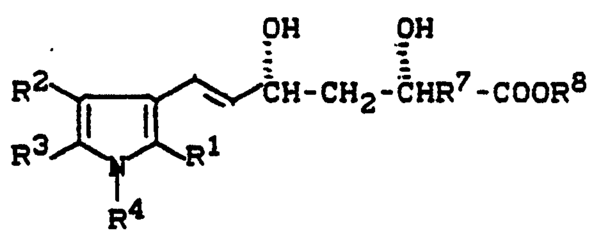

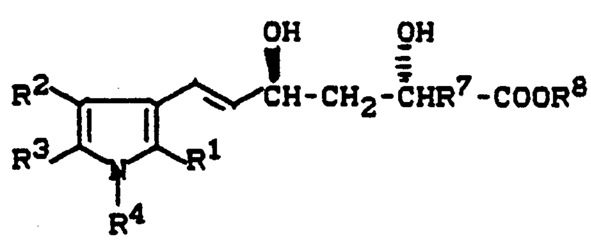

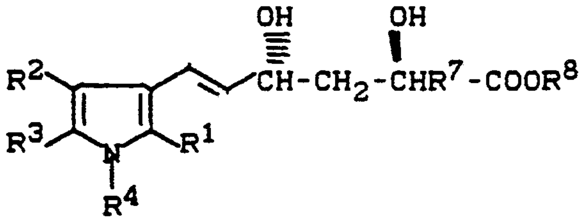

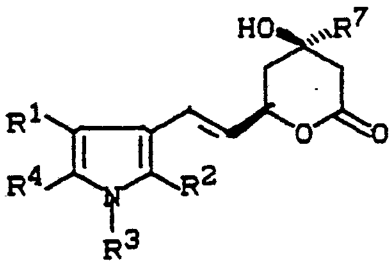

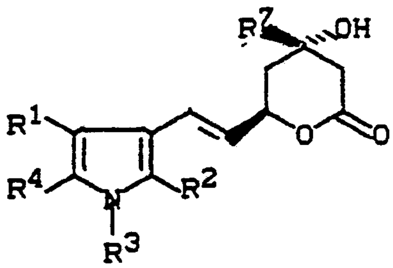

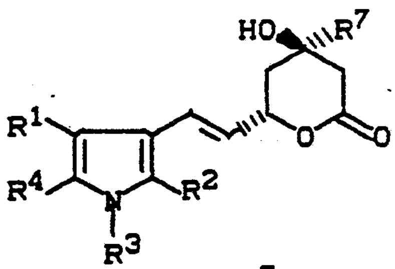

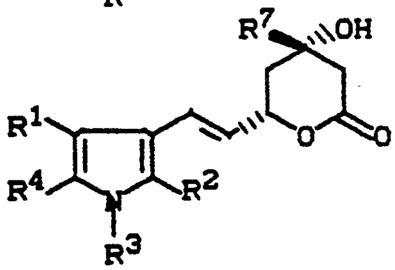

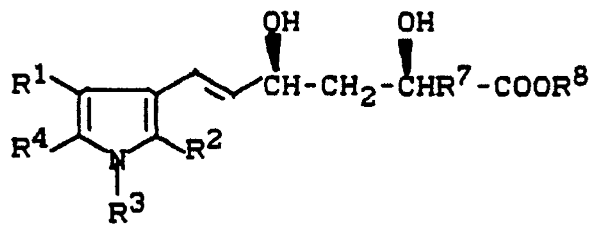

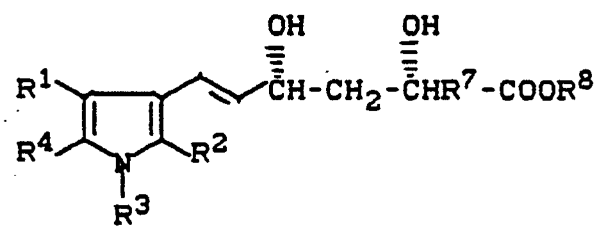

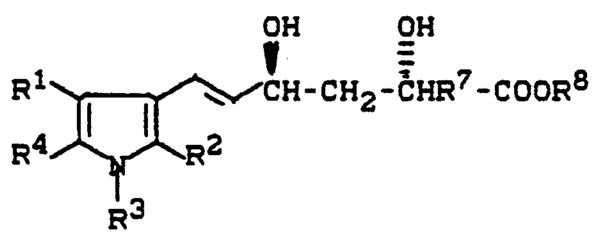

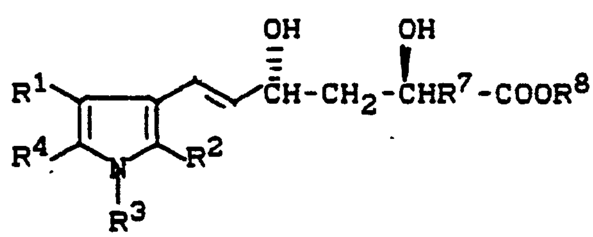

Ganz besonders bevorzugt sind die Verbindungen der allgemeinen Formel (Ia) und (Ib), in welchen

$R^1$ - für Cyclopropyl, Methyl, Ethyl, Propyl, Isopropyl, Butyl, Isobutyl oder tert.Butyl steht,

$R^2$ und $R^3$ - gleich oder verschieden sind und

-für Phenyl stehen, die bis zu 2-fach gleich oder verschieden durch Methyl, Ethyl, Propyl, Isopropyl, Methoxy, Ethoxy, Phenoxy, Benzyloxy, Fluor, Chlor oder Trifluormethyl substituiert sein können,

wobei $R^3$ auch Methyl, Propyl oder iso-Propyl sein kann,

$R^4$ -für Wasserstoff, Cyclopropyl, Cyclopentyl oder Cyclohexyl steht, oder

-für Methyl, Ethyl, Propyl, Isopropyl, Butyl, Isobutyl, tert.Butyl, Pentyl, Isopentyl, Hexyl oder Isohexyl steht,

oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy,

oder

-für Phenyl steht, das bis zu 2-fach gleich oder verschieden durch Methyl, Ethyl, Methoxy, Ethoxy, Phenyl, Benzyl, Fluor, Chlor, Trifluormethyl,

oder

X - für eine Gruppe der Formel

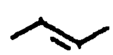 (E-konfiguriert)

steht

und
A - für eine Gruppe der Formel

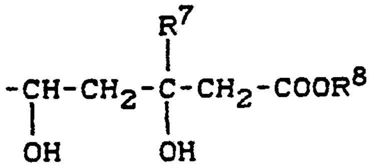

oder

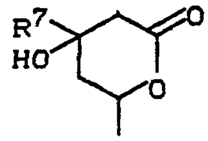

steht,
worin
$R^7$ -Wasserstoff bedeutet
und
$R^8$ -Wasserstoff, Methyl oder Ethyl bedeutet oder ein Natrium-oder Kaliumkation bedeutet.

Außerdem wurde ein Verfahren zur Herstellung der substituierten Pyrrole der allgemeinen Formel (I)

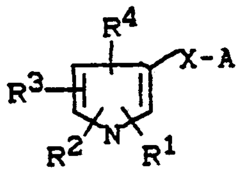

(I)

in welcher
$R^1$, $R^2$, $R^3$, $R^4$, $R^5$, $R^6$, X und A die obengenannte Bedeutung haben,
gefunden,
das dadurch gekennzeichnet ist, daß man
Ketone der allgemeinen Formel (VIII)

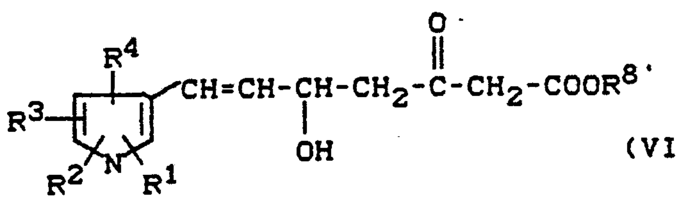

(VIII)

in welcher
$R^1$, $R^2$, $R^3$ und $R^4$ die oben angegebene Bedeutung haben, und
$R^{8'}$ -für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

Das erfindungsgemäße Verfahren kann durch das folgende Formelschema erläutert werden:

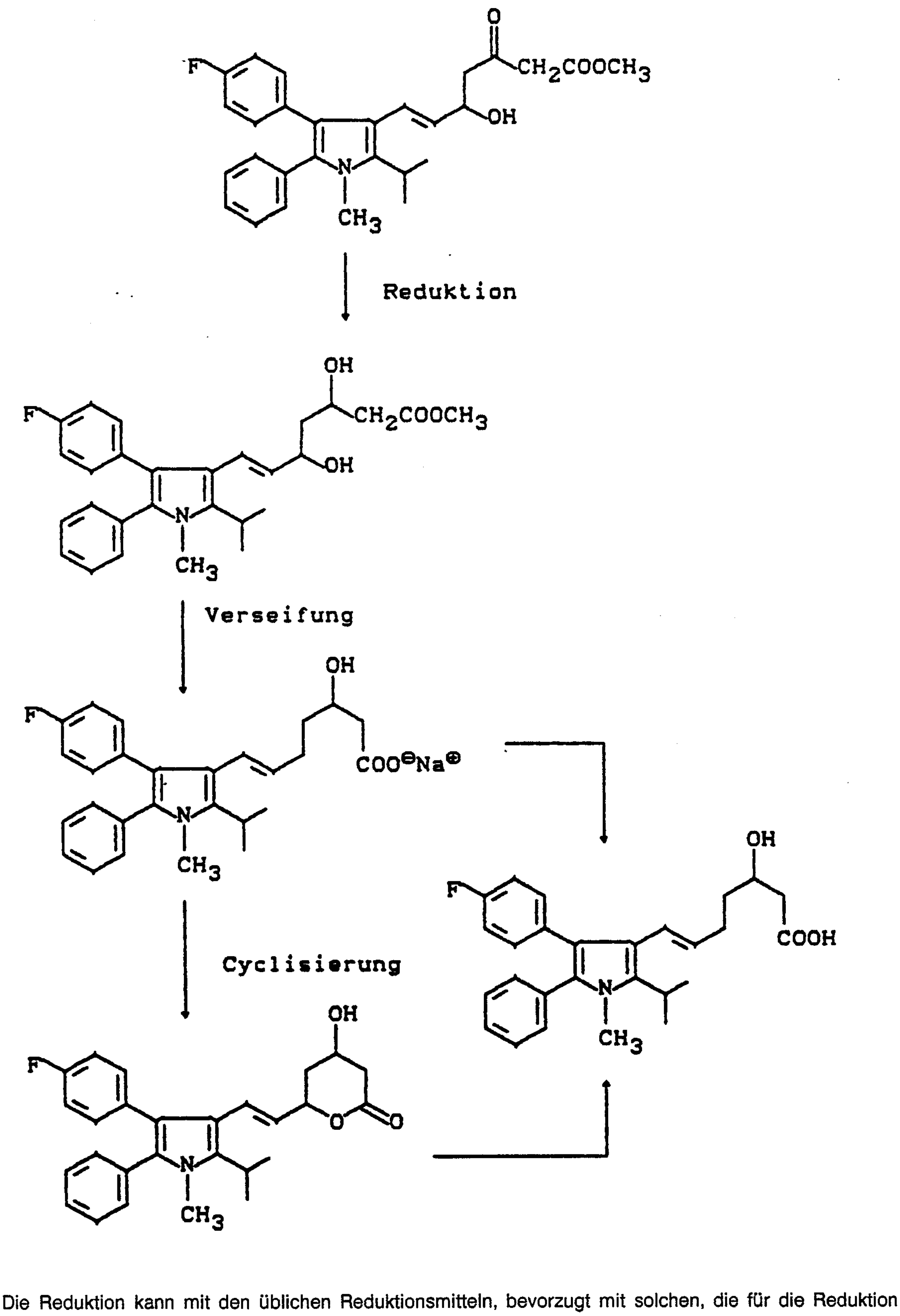


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannte Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis Raumtemperatur, bevorzugt von -78°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

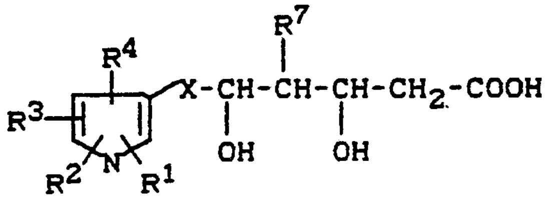

(Ic)

in welcher

$R^1$, $R^2$, $R^3$, $R^4$, $R^7$ und X die oben angegebene Bedeutung haben.

Die Carbonsäureester im Rahmen der allgemeinen Formel (I) entsprechen der Formel (Id)

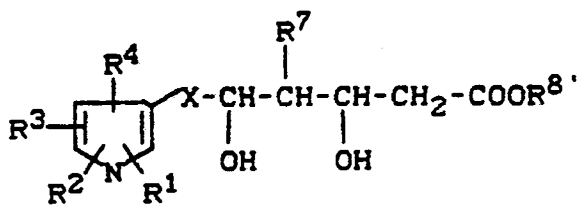

(Id)

in welcher

$R^1$, $R^2$, $R^3$, $R^4$, $R^7$ und X die oben angegebene Bedeutung haben,

und

$R^{8'}$ -für Alkyl steht.

Die Salze der erfindungsgemäßen Verbindungen im Rahmen der allgemeinen Formel (I) entsprechen der Formel (Ie)

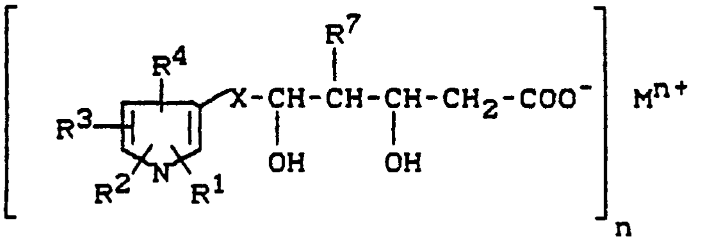

(Ie)

in welcher
$R^1$, $R^2$, $R^3$, $R^4$, $R^7$ und X die oben angegebene Bedeutung haben,
und
$M^{n+}$ für ein Kation steht.

Die Lactone im Rahmen der allgemeinen Formel (I) entsprechen der Formel (If)

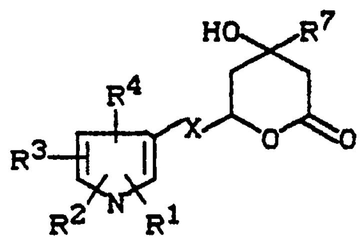

(If)

in welcher
$R^1$, $R^2$, $R^3$, $R^4$, $R^7$ und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielweise Natriumhydroxid, Kaliumhydroxid oder Bariumhydroxid, oder Alkalicarbonate wie Natrium-oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannte Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zur arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +200°C, bevorzugt von -25°C bis +50°C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cylisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N'-Dicyclohexylcarbodiimid-Paratoluolsulfonat, N-Cyclohexyl-N'-[2-(N''-methylmorpholinium)ethyl]carbodiimid oder N-(3-Dimethylaminopropyl)-N'-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen.

Die Reaktion wird im allgemeinen in einem Temperaturbereich von 0°C bis +80°C bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone oder Ester. Besonders bevoruzgt wird hierbei die racemische Mischung der erythro-Ester (IV) durch Behandeln entweder mit D-(+)-oder L-(-)-α-Methylbenzylamin nach üblichen Methoden in die diastereomeren Dihydroxyamide (Ig)

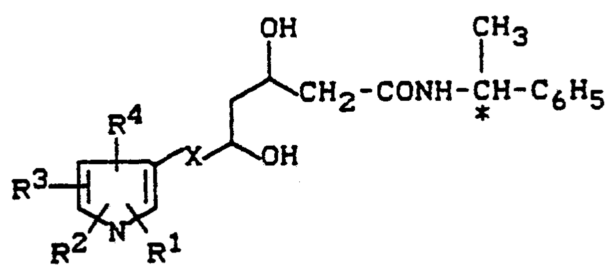

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergibt die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden:

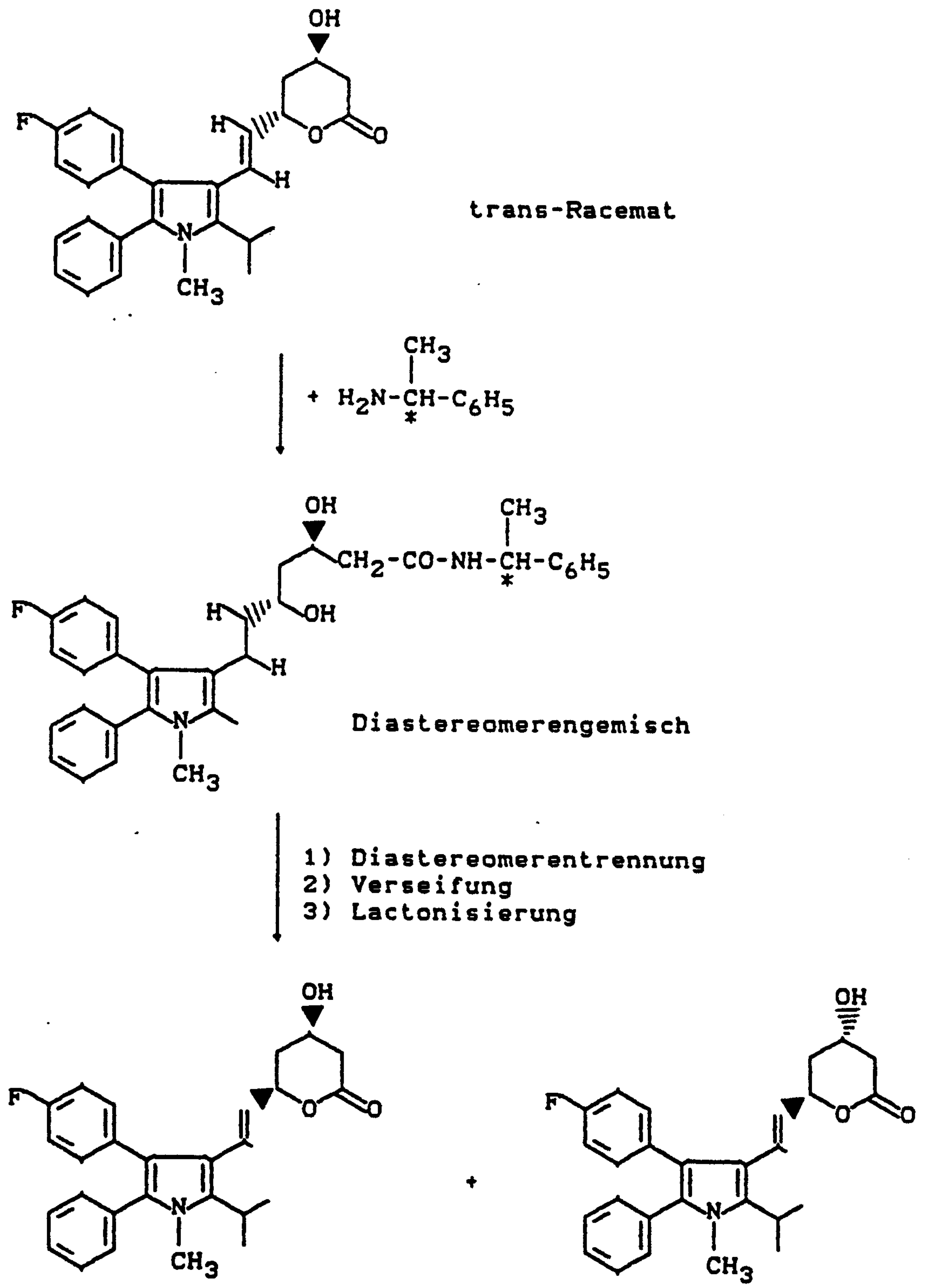


Die als Ausgangsstoffe eingesetzten Ketone (VIII) sind neu.

Es wurde ein Verfahren zur Herstellung der erfindungsgemäßen Ketone der allgemeinen Formel (VIII)

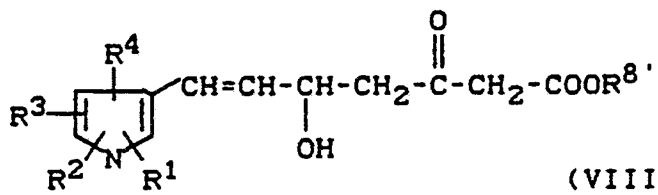

(VIII)

in welcher $R^1$, $R^2$, $R^3$, $R^4$, $R^5$, $R^6$ die obengenannte Bedeutung haben und $R^{8'}$ -für Alkyl steht, gefunden, das dadurch gekennzeichnet ist, daß man Aldehyde der allgemeinen Formel (IX)

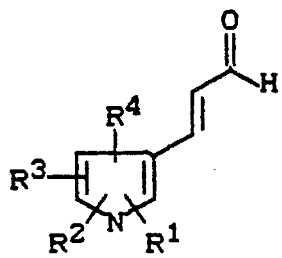

(IX)

in welcher $R^1$, $R^2$, $R^3$ und $R^4$ die oben angegebene Bedeutung haben, in inerten Lösemitteln mit Acetessigester der allgemeinen Formel (X)

$H_3C-\overset{O}{\overset{\|}{C}}-CH_2-COOR^{8'}$ (X)

in welcher $R^{8'}$ die oben angegebene Bedeutung hat, in Anwesenheit von Basen umsetzt.

Das erfindungsgemäße Verfahren kann beispielsweise durch folgendes Formelschema erläutert werden:

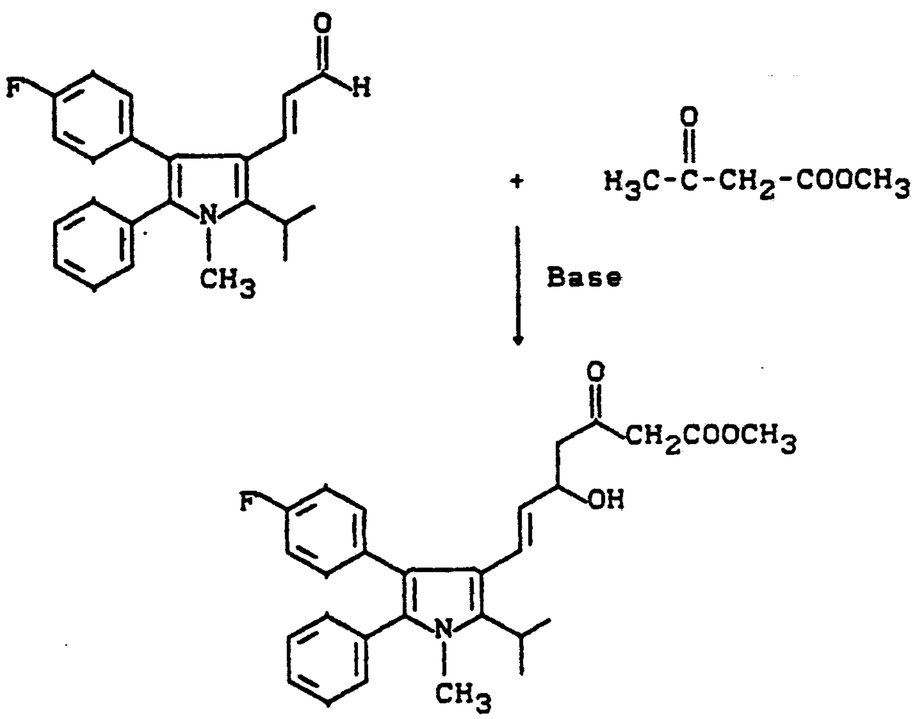

Als Basen kommen hierbei die üblichen stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropyl amid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdrück oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

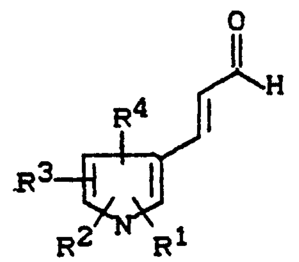

(IX)

in welcher
$R^1$, $R^2$, $R^3$, $R^4$, $R^5$ und $R^6$ die oben genannte Bedeutung haben,
gefunden, das dadurch gekennzeichnet ist, daß man
Pyrrole der allgemeinen Formel (XI)

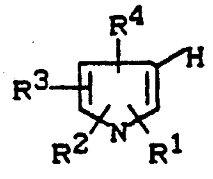

(XI)

in welcher
$R^1$, $R^2$, $R^3$ und $R^4$ die oben angegebene Bedeutung haben,
in inerten Lösemitteln in Anwesenheit von Hilfsstoffen mit N,N-Dimethylaminoacrolein der Formel (XII)

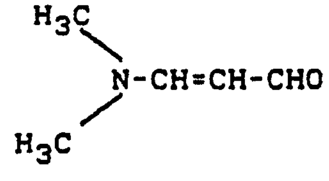

(XII)

umsetzt.

Das erfindungsgemäße Verfahren kann beispielsweise durch folgendes Formelschema verdeutlicht werden:

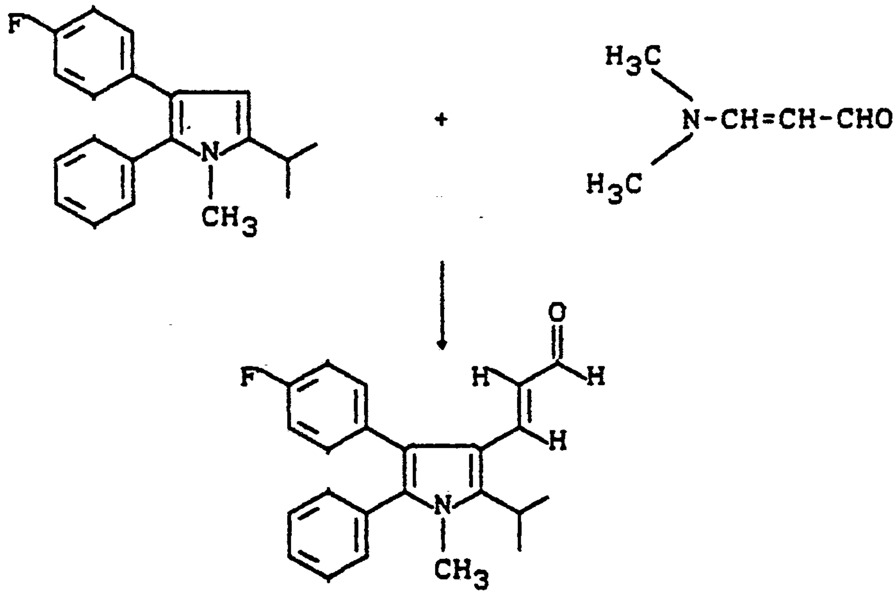

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder Dichlorobenzol, oder Ether wie Diethylether, Dioxan oder Tetrahydrofuran, oder Chlorkohlenwasserstoffe wie Methylenchlorid, Chloroform oder Tetrachlorkohlenstoff, oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20°C bis +150°C, bevorzugt von 0°C bis +100°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgeinen in einer Menge von 2 bis 6, bevorzugt von 3 bis 4 mol, bezogen auf 1 mol des Pyrrols eingesetzt.

Die als Ausgangsstoffe eingesetzten Pyrrole der allgemeinen Formel (XI) sind bekannt oder können nach bekannten Methoden hergestellt werden [A. Glossauer "Die Chemie der Pyrrole", Springer Verlag Berlin, 1974].

Die efindungsgemäßen Verbindungen der allgemeinen Formel (I) besitzen wertvolle pharmakologische Eigenschaften, insbesondere sind sie Inhibitoren der 3-Hydroxy-3-methyl-glutarylCoenzym A (HGM-CoA) Reduktase und infolge dessen Inhibitoren der Cholesterolbiosynthese. Sie können deshalb zur Behandlung von Hyperlipoproteinämie, Lipoproteinämie oder Artheriosklerose eingesetzt werden. Die erfindungsgemäßen Wirkstoffe bewirken außerdem eine Senkung des Chloesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegenbenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

1-(4-Fluorphenyl)-4-methyl-pent-1-en-3-on

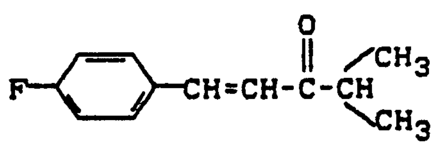

Zu 198,4 g (1,6 mol) frisch destilliertem 4-Fluorbenzaldehyd und 137,6 g (1,6 mol) Methylisopropylketon in 300 ml Methanol tropft man 75 ml 15%ige Kalilauge und rührt über Nacht bei Raumtemperatur. Dann wird mit 10 ml Essigsäure neutralisiert, 1 l Wasser zugesetzt und mit zwei 500 ml Portionen Ether extrahiert. Die vereinigten organischen Phasen werden mit 500 ml gesättigter Natriumchlorid-Lösung gewaschen und über Natriumsulfat getrocknet. Nach Abziehen des Lösemittels destilliert man im Hochvakuum.
Ausbeute: 198,6 g (65% der Theorie) gelbliches Öl
Kp.: 103°C (0.3 mbar)
$^1$H-NMR ($CDCl_3$): $\delta$ = 1,2 (d, 6H, $CH_3$); 2,9 (sept, 1H, CH-($CH_3$)$_2$); 6,8 (d, 1H, Olefin-H); 7,1 (m, 2H, Aromaten-H); 7,6 (m, 3H, Aromaten-H + Olefin-H).

Beispiel 2

2-(4-Fluorphenyl)-5-methyl-1-phenyl-hexan-1,4-dion

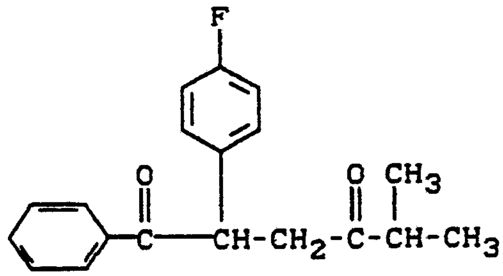

Zu einer Lösung von 5,88 g (0,12 mol) Natriumcyanid in 300 ml Dimethylformamid, tropft man bei 35°C eine Lösung von 63,6 g (0,6 mol) frisch destilliertem Benzaldehyd in 300 ml Dimethylformamid innerhalb von 30 min und rührt weitere 5 min bei dieser Temperatur. Dann werden innerhalb von 1,5 h 86,5 g (0,45 mol) 1-(4-Fluorphenyl)-4-methyl-pent-1-en-3-on (Beispiel 1) in 500 ml Dimethylformamid zugetropft und 1 h nachgerührt, wobei die Temperatur stets bei 35°C gehalten wird. Nach Versetzen mit 1 l Wasser wird viermal mit je 400 ml Chloroform extrahiert, die vereinigten organischen Phasen mit 1 l gesätti. er Natriumhydrogencarbonat-Lösung und 1 l Wasser gewaschen und über Natriumsulfat getrocknet. Nach dem Einengen im Vakuum destilliert man unter vermindertem Druck bis zuletzt eine Fraktion bei 138 - 142°C (0,9 mbar) übergeht. Der Destillationsrückstand (132 g) wird nun in zwei Portionen an einer Säule (1,5 Kg Kieselgel 230 - 400 mesh, Ø 9 cm), mit Petrolether / Essigester (10 : 1) chromatographiert. Das Produkt wird nach 4 - 7 l Elutionsmittel erhalten. Nach Abziehen des Lösemittels bleiben 90,0 g (67% der Theorie), farbloses Öl.

$^1$H-NMR ($CDCl_3$): $\delta$ = 1,08 (d, 3H, $CH_3$); 1,12 (d, 3H, $CH_3$); 2,65 (sept, 1H, $\underline{CH}$-$(CH_3)_2$); 2,7 (dd, 1H, -CO-$\underline{CH_2}$-CH); 3,6 (dd, 1H, -CO-$CH_2$-CH); 5,12 (dd, 1H, H-C-$C_6H_4$-F); 6,95 (m, 2H, Aromaten-H); 7,23 (m, 2H, Aromaten-H); 7,4 (m, 3H, Aromaten-H); 7,95 (m, 2H, Aromaten-H).

<u>Beispiel 3</u>

3-(4-Fluorphenyl)-5-isopropyl-1-methyl-2-phenyl-pyrrol

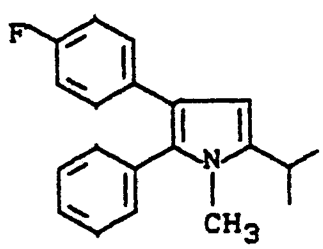

2,98 g (10 mmol) 2-(4-Fluorphenyl)-5-methyl-1-phenyl-hexan-1,4-dion (Beispiel 2) werden in 10 ml 40%iger wäßriger Methylaminlösung auf 80°C erwärmt und mit ca. 14 ml Ethanol versetzt, so daß eine klare Lösung entsteht. Man erhitzt noch 30 min zum Rückfluß und saugt nach dem Abkühlen den Niederschlag ab. Dieser wird mit wenig Ethanol gewaschen und im Vakuumexsiccator über Phosphorpentoxid getrocknet.

Ausbeute: 1,8 g (61% der Theorie) farblose Kristalle

Schmp.: 114°C

$^1$H-NMR ($CDCl_3$): $\delta$ = 1,35 (d, 6H, CH-$\underline{CH_3})_2$); 3,9 (sept, 1H, $\underline{CH}$-$(CH_3)_2$); 3,42 (s, 3H, N-$CH_3$); 6,17 (s, 1H, pyrrol-H); 6,8 - 7,4 (m, 9H, Aromaten-H).

Beispiel 4

(E)-3-[3-(4-Fluorphenyl)-5-isopropyl-1-methyl-2-phenyl-pyrrol-4-yl]prop-2-enal

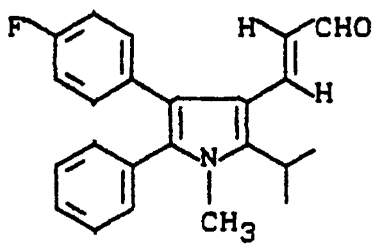

1,64 ml (18 mmol) Phosphoroxychlorid werden in 10 ml wasserfreien Acetonitril vorgelegt. Unter Argon tropft man zuerst bei -10° bis 0°C eine Lösung von 1,85 g (16,8 mmol) 90%iges Dimethylaminoacrolein in 7 ml Acetonitril und dann bei -5°C bis 0°C eine Lösung von 1,76 g (6 mmol) 3-(4-Fluorphenyl)-5-isopropyl-1-methyl-2-phenyl-pyrrol (Beispiel 3) in 7 ml Acetonitril zu. Über Nacht wird im Rückfluß erhitzt. Den Ansatz gibt man nun bei 10°C in die Suspension aus 4,4 g Natriumhydroxid in 65 ml Wasser und 65 ml Toluol, so daß die Temperatur 25°C nicht übersteigt. Nach 1,5 h Rühren bei Raumtemperatur wird über Kieselgur filtriert und die Phasen getrennt.

Nun wird die organische Phase über Natriumsulfat getrocknet, eingeengt und an einer Säule (100 g Kieselgel 230 - 400 mesh, ⌀ 4 cm, Methylenchlorid) chromatographiert. Das gereinigte Produkt kocht man schließlich in Methanol aus.

Ausbeute: 1,3 g (62% der Theorie) gelblicher Feststoff

Schmp.: 215°C

$^1$H-NMR ($CDCl_3$): δ = 1,5 (d, 6H, CH-($CH_3$)$_2$); 3,5 (m, 4H, N-$CH_3$ + CH-($CH_3$)$_2$); 5,8 (dd, 1H, Olefin-H); 6.8 - 7,3 (m, 9H, Aromaten-H); 7,6 (d, 1H, Olefin-H); 9,4 (d, 1H, CHO).

Beispiel 5

Methyl-(E)-7-[3-(4-fluorphenyl)-5-isopropyl-1-methyl-2-phenyl-pyrrol-4-yl]-5-hydroxy-3-oxo-hept-6-enoat

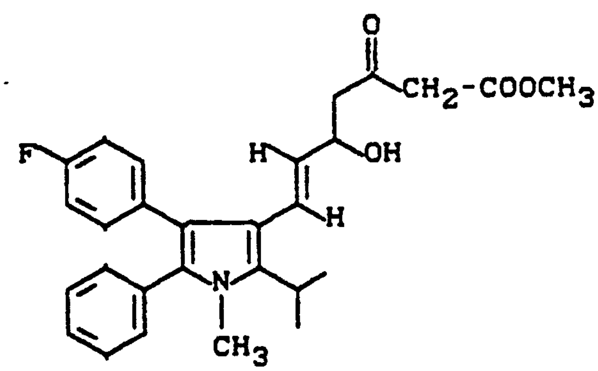

Unter Argon tropft man zu einer Suspension von 0,11 g (3,6 mmol) 80%ige Natriumhydrid in 10 ml trockenem Te trahydrofuran bei 0°C 0,38 (3,3 mmol) Acetessigsäuremethylester. Nach 15 min werden bei derselben Temperatur innerhalb von 10 min 2,1 ml 15%igem Butyllithium in Hexan zugetropft und 15 min nachgerührt. Jetzt tropft man eine Lösung von 1,04 g (3 mmol) (E)-3-[3'-(4"-Fluorphenyl)-5'-isopropyl-1'-methyl-2'-phenyl-pyrrol-4'-yl]-prop-2-enal (Beispiel 4) in 15 ml trockenem Tetrahydrofuran bei 0°C zu und rührt weitere 15 min bei dieser Temperatur. Anschließend werden vorsichtig 15 ml 0,6 N Salzsäure

zugegeben, die Mischung dreimal mit je 15 ml Ether extrahiert, die vereinigten organischen Phasen mit gesättigter Natriumchlorid-Lösung gewaschen und über Natriumsulfat getrocknet. Nach dem Einengen im Vakuum wird der Rückstand in einer Säule (50 g Kieselgel 230 - 400 mesh, ⌀ 3 cm) mit Toluol / Essigester (5 : 1) chromatographiert.

Ausbeute: 0,58 g (42% der Theorie) gelbliches Öl.

$^1$H-NMR ($CDCl_3$): $\delta$ = 1,45 (d, 6H, CH-($CH_3$)$_2$); 2,65 (m, 2H, -CH(OH)-$CH_2$-CO-); 3,45 (m, 5H, N-$CH_3$, $CH_2$-$COCH_3$); 3,73 (s, 3H, O-$CH_3$); 4,58 (m, 1H, HO-C-H); 5,2 (dd, 1H, Olefin-H); 6,0 (d, 1H, Olefin-H); 6,8 - 7,3 (m, 9H, Aromaten-H).

Beispiel 6

Methyl-erythro-(E)-7-[3-(4-fluorphenyl)-5-isopropyl-1-methyl-2-phenyl-pyrrol-4-yl]-3,5-dihydroxy-hept-6-enoat

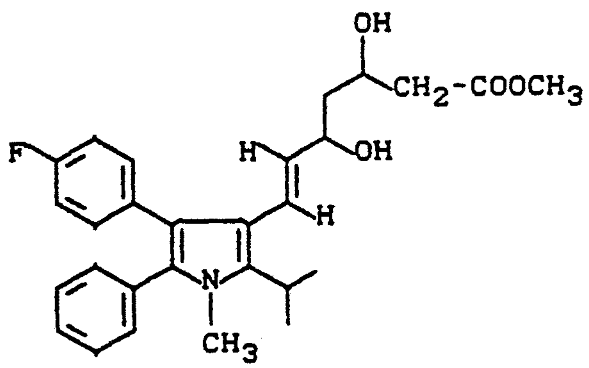

Zu einer Lösung von 465 mg (1 mmol) Methyl-(E)-7-[3′-(4″-fluorphenyl)-5′-isopropyl-1′-methyl-2′-phenyl-pyrrol-4′-yl]-5-hydroxy-3-oxo-hept-6-enoat (Beispiel 5) in 15 ml trockenem Tetrahydrofuran tropft man bei Raum temperatur unter Argon 1,2 ml (1,2 mmol) 1M Triethylboran-Lösung in Hexan, leitet während 5 min Luft durch die Lösung und kühlt dann auf -78°C ab.

Es werden 47 mg (1,24 mmol) Natriumborhydrid und langsam 0,6 ml Methanol zugegeben, 15 min bei -78°C und 15 min bei -30°C gerührt.

Bei 0°C tropft man dann eine Lösung von 3,3 ml 30%iges Wasserstoffperoxid in 7 ml Wasser zu, läßt auf Raumtemperatur erwärmen, extrahiert mit 15 ml Essigester, wäscht die organische Phase mit je 10 ml 0,5 N Salzsäure und gesättigter Natriumchlorid-Lösung und engt unter vermindertem Druck ein. Der Rückstand (450 mg) wird an 80 g Kieselgel 230 - 400 mesh, in einer Säule von 4 cm Durchmesser mit Petrolether / Essigester (1:1) chromatographiert.

Ausbeute: 240 mg (52% der Theorie) gelbliches Öl

MS: m/e 465 (5%, $M^+$), 447 (80%, M-$H_2O$)

$^1$H-NMR ($CDCl_3$): $\delta$ = 1,45 (d, 6H, CH-($CH_3$)$_2$); 1,6 (m, 2H, CH(OH)-$CH_2$-CHOH); 2,45 (m, 2H, $CH_2$-$CO_2$-$CH_3$); 2,6 (d, 1H, OH); 3,4 (sept, 1H, CH-($CH_3$)$_2$); 3,5 (s, 3H, N-$CH_3$); 3,6 (d, 1H, OH); 3,7 (s, 3H, O-$CH_3$); 4,2 (m, 1H, OH-CH); 4,4 (m, 1H, HO-CH); 5,2 (dd, 1H, Olefin-H); 6,6 (d, 1H, Olefin-H); 6,8 - 7,3 (m, 9H, Aromaten-H).

Beispiel 7

1-(4-Fluorphenyl)-4-methyl-pent-2-en-1-on

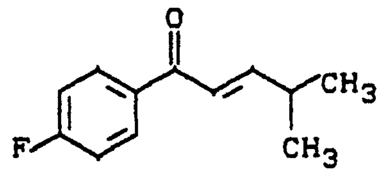

Zu 138,1 g (1,0 mol) 4-Fluoracetophenon gibt man eine Lösung von 17 g (0,3 mol) Kaliumhydroxid in 125 ml Wasser/125 ml Methanol, erwärmt auf 50°C und tropft innerhalb 1,5 h 80 g (1,1 mol) 2-Methylpropanal zu, wobei ein Feststoff ausfällt. Nach weiteren 3,5 h Rühren bei 50°C läßt man auf 25°C abkühlen, saugt den Feststoff ab, wäscht ihn mit 150 ml Methanol und erhält nach Trocknen 132,3 g dimeres Kondensationsprodukt. Der Feststoff wird mit 5 g Natriumacetat vermischt und im Wasserstrahlvakuum über eine 20 cm-Vigreuxkolonne destilliert. Man erhält ein Gemisch (1:1,8) aus 1-(4-Fluorphenyl)-4-methyl-pent-2-en-1-on und 1-(4-Fluorphenyl)-4-methyl-pent-3-en-1-on.
Ausbeute: 120,2 g (62% der Theorie)
Kp.: 132°C (14 mbar)
$^1$H-NMR ($CDCl_3$): $\delta$ = 1,15 (d, 6H, $CH_3$); 1,7 (s, 3H, $CH_3$); 1,8 (s, 3H, $CH_3$); 2,6 (sept, 1H, $\underline{CH}$-$(CH_3)_2$); 3,7 (d, 2H, $\underline{CH_2}$-CH=C$(CH_3)_2$); 5,4 (m, 1H, $CH_2$-$\underline{CH}$=C$(CH_3)_2$); 6,7 (d, 1H, Olefin-H); 7,1 (dd, 1H, Olefin-H); 7,2 (m, 4H, Aromaten-H); 8,0 (m, 4H, Aromaten-H).

## Beispiel 8

1-(4-Fluorphenyl)-4-methyl-3-(1-nitroethyl)-pentan-1-on

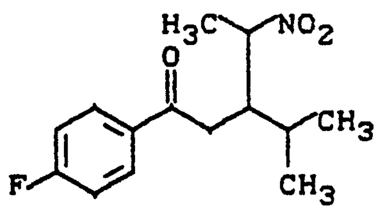

Zu einer Lösung von 50 g (0,26 mol) des Isomerengemisches (1:1,8) aus Beispiel 7 und 25 g (0,3 mol) Nitroethan in 150 ml Acetonitril tropft man bei 0°C 18,9 g (0,13 mol) 1,5-Diazabicyclo(5,4,0)undec-5-en in 100 ml Acetonitril. Nach 2 h Rühren bei 25°C wird mit 260 ml 1N-Salzsäure versetzt und dreimal mit Dichlormethan extrahiert. Die vereinigten organischen Phasen werden mit 130 ml 1N-Salzsäure, Natriumhydrogencarbonatlösung und Wasser gewaschen, über Natriumsulfat getrocknet und im Vakuum eingeengt. Man erhält 63 g eines Öls, das über 800 g Kieselgel mit Petrolether/Essigester 10:1 chromatographiert wird.
Ausbeute: 31 g (44% der Theorie) Öl als Diastereomerengemisch.

## Beispiel 9

1-(4-Fluorphenyl)-3-isopropyl-pentan-1,4-dion

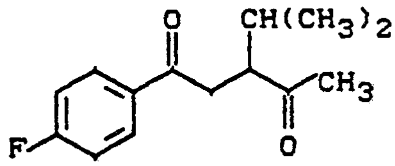

Zu einer Lösung von 18,7 g (70 mmol) Beispiel 8 in 120 ml Ethanol gibt man 40 ml 2 N Natronlauge und tropft diese Lösung bei 0°C in 64 ml 3 N Schwefelsäure. Nach 1 h Rühren bei Raumtemperatur wird das Ethanol im Vakuum entfernt, der Rückstand in Wasser/Dichlormethan aufgenommen, dreimal mit Dichlormethan extrahiert, die vereinigten organischen Phasen mit 1 N Salzsäure sowie gesättigter Natriumhydrogencarbonatlösung und Wasser gewaschen. Die über Natriumsulfat getrocknete Lösung wird im Vakuum eingeengt und das erhaltene Öl (18,4 g) über Kieselgel mit Petrolether / Essigester (10:1) chromatographiert.
Ausbeute: 16 g (97% der Theorie)
$^1$H-NMR ($CDCl_3$): $\delta$ = 0,9 (d, 3H, $CH_3$); 1,0 (d, 3H, $CH_3$); 2,1 (sept, 1H, $\underline{CH}(CH_3)_2$); 2,3 (s, 3H, $CH_3C=O$); 2,9 (dd, 1H, $CH_2$-C=O); 3,1 (m, 1H, CH-C=O); 3,5 (dd, 1H, $CH_3$-C=O); 7,1 (m, 2H, Aromaten-H); 8,0 (m, 2H, Aromaten-H).

Beispiel 10

2-(4-Fluorphenyl)-5-methyl-1-phenyl-4-isopropyl-pyrrol

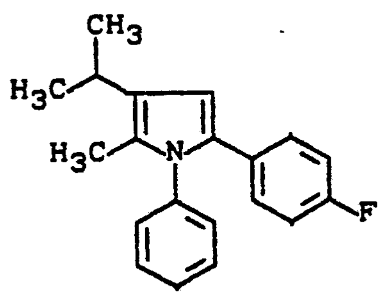

Eine Lösung von 25,3 g (0,11 mol) Beispiel 9, 70 g (0,75 mol) Anilin und eine Spatelspitze p-Toluolsulfonsäure in Toluol werden über Nacht am Wasserabscheider unter Rückfluß erhitzt. Das Lösemittel wird anschließend im Vakuum entfernt und der Rückstand (20,2 g) aus Petrolether umkristallisiert.
Ausbeute: 7,1 g (22,5% der Theorie)
$^1$H-NMR ($CDCl_3$): $\delta$ = 1,3 (d, 6H, $(\underline{CH_3})_2CH$); 2,1 (s, 3H, $CH_3$); 2,9 (sept, 1H, $\underline{CH}(CH_3)_2$); 6,3 (s, 1H, Pyrrol-H); 6,8 (m, 2H, Aromaten-H); 7,0 (m, 2H, Aromaten-H); 7,1 (m, 2H, Aromaten-H); 7,4 (m, 3H, Aromaten-H).

Beispiel 11

(E)-3-[2-(4-Fluorphenyl)-5-methyl-1-phenyl-4-isopropyl-pyrrol-3-yl]-prop-2-enal

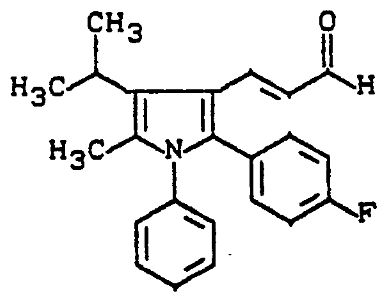

Die oben genannte Verbindung wird analog Beispiel 4 aus der Verbindung aus Beispiel 10 erhalten.
Ausbeute: 82% der Theorie
$^1$H-NMR ($CDCl_3$): δ = 1,4 (d, 6H, ($\underline{CH_3}$)$_2$CH); 2,1 (s, 3H, $CH_3$); 3,3 (sept, 1H, $\underline{CH}(CH_3)_2$); 6,2 (dd, 1H, Olefin-H); 6,9 (m, 2H, Aromaten-H); 7,0 (m, 4H, Aromaten-H); 7,3 (m, 3H, Aromaten-H); 7,4 (d, 1H, Olefin-H); 9,4 (d, 1H, CHO).

Beispiel 12

Methyl-(E)-7-[2-(4-fluorphenyl)-5-methyl-1-phenyl-4-isopropyl-pyrrol-3-yl]-5-hydroxy-3-oxo-hept-6-enoat

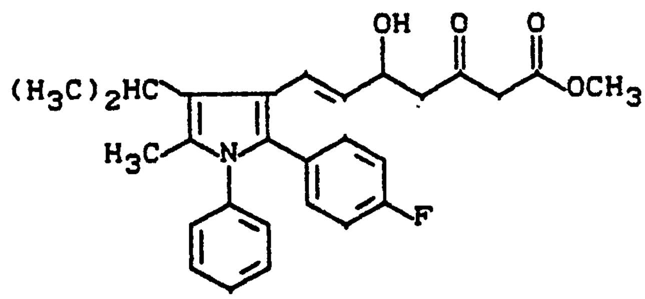

Die oben genannte Verbindung wird Analog Beispiel 5 aus der Verbindung aus Beispiel 11 erhalten.
Ausbeute: 0,7 g (35% der Theorie)
$^1$H-NMR ($CDCl_3$): δ = 1,4 (d, 6H, ($\underline{CH_3}$)$_2$CH); 2,1 (s, 3H, $CH_3$); 2,7 (m, 2H, CH(OH)$\underline{CH_2}$); 3,2 (sept, 1H, $\underline{CH}$-($CH_3$)$_2$); 3,5 (s, 2H, $\underline{CH_2}COOCH_3$); 3,7 (s, 3H, $OCH_3$); 4,6 m, 1H, CHOH); 5,4 (dd, 1H, Olefin-H); 6,5 (d, 1H, Olefin-H); 6,8 (m, 2H, Aromaten-H); 7,0 (m, 2H, Aromaten-H); 7,2 (m, 5H, Aromaten-H).

Beispiel 13

Methyl-erythro-(E)-7-[2-(4-fluorphenyl)-5-methyl-1-phenyl-4-isopropyl-pyrrol-3-yl]-3,5-dihydroxy-hept-6-enoat

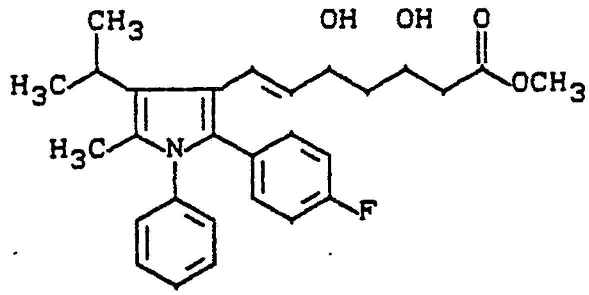

Die oben genannte Verbindung wird analog Beispiel 6 aus der Verbindung aus Beispiel 12 erhalten.
Ausbeute: 0,42 g (69% der Theorie)
$^1$H-NMR ($CDCl_3$): δ = 1,4 (d, 6H, CH($CH_3$)$_2$); 1,6 (m, 2H, $CH_2$-CHOH); 2,1 (s, 3H, $CH_3$); 2,5 (m, 2H, $CH_2COOCH_3$); 3,2 (sept, 1H, CH-($CH_3$)$_2$); 3,7 (s, 3H, $OCH_3$); 4,2 (m, 1H, CHOH); 4,4 (m, 1H, CHOH); 5,4 (dd, 1H, Olefin-H); 6,5 (d, 1H, Olefin-H); 6,8 - 7,3 (m, 9H, Aromaten-H).

Beispiel 14

Methyl-erythro-(E)-7-[1-(2,6-dimethylphenyl)-2-(4-fluorphenyl)-5-methyl-4-isopropyl-pyrrol-3-yl]-3,5-dihydroxy-hept-6-enoat

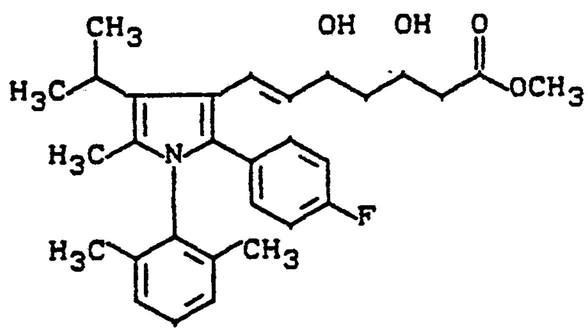

Die oben genannte Verbindung wird analog Beispiel 13 erhalten.
Ausbeute: 67% der Theorie
$^1$H-NMR ($CDCl_3$): δ = 1,4 (d, 6H, ($CH_3$)$_2$CH); 1,6 (m, 2H, $CH_2$-CHOH); 2,0 (m, 9H, $CH_3$); 2,5 (m, 2H, $CH_2COOCH_3$); 3,2 (m, 1H, OH-($CH_3$)$_2$); 3,7 (s, 3H, $OCH_3$); 4,2 (m, 1H, CHOH); 4,4 (m, 1H, CHOH); 5,5 (dd, 1H, Olefin-H); 6,5 (d, 1H, Olefin-H); 6,7 - 7,3 (m, 7H, Aromaten-H).

Beispiel 15

Methyl-erythro-(E)-7-[1-benyl-2-(4-fluorphenyl)-4-isopropyl-5-methyl-pyrrol-3-yl]-3,5-dihydroxy-hept-6-enoat

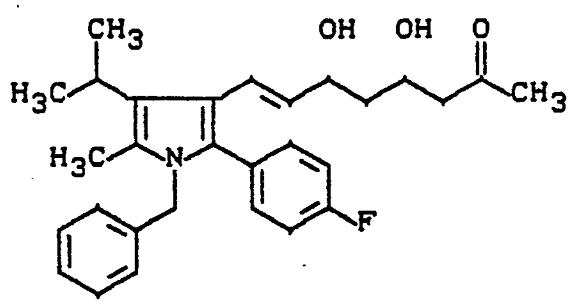

Die oben genannte Verbindung wird analog Beispiel 13 erhalten.
Ausbeute: 39% der Theorie
$^1$H-NMR ($CDCl_3$): $\delta$ = 1,3 (d, 6H, ($\underline{CH_3}$)$_2$CH); 1,6 (m, 2H, $\underline{CH_2}$CHOH); 2,1 (s, 3H, $CH_3$); 2,5 (m, 2H, $\underline{CH_2}COOCH_3$); 3,1 (m, 1H, $\underline{CH}(CH_3)_2$); 3,7 (s, 3H, $OCH_3$); 4,2 (m, 1H, CHOH); 4,3 (m, 1H, CHOH); 4,9 (s, 2H, $CH_2Ph$); 5,3 (dd, 1H, Olefin-H); 6,5 (d, 1H, Olefin-H); 6,7 - 7,3 (m, 9H, Aromaten-H).

In Analogie zu Beispiel 15 und den in den Beispielen 1-5 beschriebenen Reaktionen wurden die in Tabelle 1 aufgeführten Verbindungen erhalten.

Tabelle 1

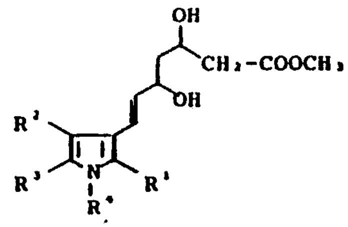

| Bsp.-Nr. | $R^1$ | $R^2$ | $R^3$ | $R^4$ | $^1$H-NMR [δ ppm, $CDCl_3$, 300 MHz] |
|---|---|---|---|---|---|
| 16 | $-CH(CH_3)_2$ | 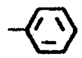 | 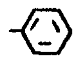 | $-CH_3$ | 1,43 (d,6H); 1,6 (m,2H); 2,47 (m,2H); 3,39 (sept.,1H); 3,47 (s,3H); 3,72 (s, 3H); 4,20 (m,1H); 4,38 (m,1H); 5,24 (dd, (1H); 6,60 (d,1H); 7,0 - 7,3 (m,10H). |
| 17 | $-CH(CH_3)_2$ | 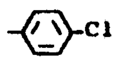 | 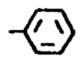 | $-CH_3$ | 1,43 (d,6H); 1,6 (m,2H); 2,47 (m,2H); 3,34 (m,1H); 3,45 (s,3H); 3,72 (s,3H); 4,23 (m,1H); 4,38 (m,1H); 5,36 (dd,1H); 6,56 (d,1H); 6,90 - 7,30 (m,9H). |
| 18 | $-CH(CH_3)_2$ | 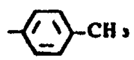 | 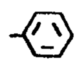 | $-CH_3$ | 1,43 (d,6H); 1,6 (m,2H); 2,26 (s,3H); 2,47 (m,2H); 3,38 (sept.,1H); 3,47 (s,3H); 3,72 (s,3H); 4,22 (m,1H); 4,38 (m,1H); 5,28 (dd,1H); 6,57 (d,1H); 6,90 - 7,30 (m, 9H). |
| 19 | $-CH(CH_3)_2$ | 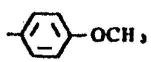 | 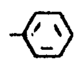 | $-CH_3$ | 1,43 (d, 6H); 1,6 (m;2H); 2,47 (m,2H); 3,38 (sept.,1H); 3,47 (s,3H); 3,71 (s, 3H); 3,76 (s,3H); 4,22 (m,1H); 4,37 (s, 1H); 5,27 (dd, 1H); 6,57 (d, 1H); 6,70 - 7,30 (m,1H). |
| 20 | $-CH(CH_3)_2$ | 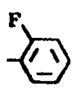 | 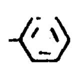 | $-CH_3$ | 1,43 (d,6H); 1,6 (m,2H); 2,44 (m,2H); 3,35 (sept.,1H); 3,48 (s,3H); 3,68 (s, 3H); 4,15 (m,1H); 4,35 (m,1H); 5,12 (dd, 1H); 6,62 (d, 1H); 6,9 - 7,3 (m, 9H). |

Fortsetzung Tabelle 1

| Bsp.-Nr. | $R^1$ | $R^2$ | $R^3$ | $R^4$ | $^1H$-NMR [δ ppm, $CDCl_3$, 300 MHz] |
|---|---|---|---|---|---|
| 21 | $-CH(CH_3)_2$ | 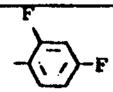 | 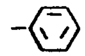 | $-CH_3$ | 1,43 (d,6H); 1,6 (m,2H); 2,47 (m,2H); 3,36 (sept.,1H); 3,48 (s,3H); 3,70 (s, 3H); 4,18 (m,1H); 4,35 (m,1H); 5,12 (dd, 1H); 6,61 (d,1H); 6,70 - 7,30 (m,8H). |
| 22 | $-CH(CH_3)_2$ | 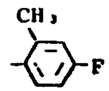 | 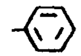 | $-CH_3$ | 1,43 (d,6H); 1,6 (m,2H); 1,98 (s,3H); 2,43 (m,2H); 3,34 (sept., 1H); 3,50 (s, 3H); 3,72 (s,3H); 4,11 (m,2H); 4,28 (m,2H); 4,94 (dd, 1H); 6,60 (d,1H); 6,70 - 7,30 (m,8H). |
| 23 | $-CH(CH_3)_2$ | 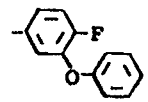 | 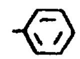 | $-CH_3$ | 1,42 (d,6H); 1,6 (m,2H); 2,45 (m,2H); 3,34 (sept.,1H); 3,43 (s,3H); 3,72 (s, 3H); 4,22 (m,1H); 4,38 (m,1H);5,27 (dd, 1H); 6,55 (d,1H); 6,70 - 7,35 (m,13H). |
| 24 | $-C(CH_3)_3$ | 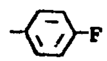 | 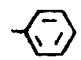 | $-CH_3$ | 1,35 (m,2H); 1,52 (s,9H); 2,40 (m,2H); 3,57 (s,3H); 3,71 (s,3H); 4.10 (m,1H); 4.32 (m,1H); 4,95 (dd,1H); 6,70 (d,1H); 6,70 - 7,30 (m,9H). |
| 25 | 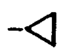 | 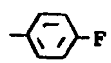 | 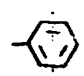 | $-CH_3$ | 0,71 (m,2H); 1,08 (m,2H); 1,67 (m,3H); 2,48 (m,2H); 3,57 (s,3H); 3,71 (s,3H); 4,27 (m,1H); 4,42 (m,1H); 5,63 (dd,1H); 6,52 (d,1H); 6,80 - 7,30 (m,9H). |
| 26 | $-CH(CH_3)_2$ | 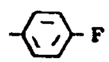 | 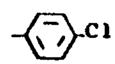 | $-CH_3$ | 1,43 (d,6H); 1,6 (m,2H); 2,47 (m,2H); 3,36 (sept.1H); 3,47 (s,3H); 3,72 (s, 3H); 4,21 (m,1H); 4,36 (m,1H); 5,20 (dd, 1H); 6,55 (d,1H); 6,80 - 7,20 (m,8H). |
| 27 | $-CH(CH_3)_2$ | 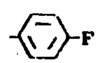 | 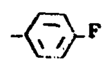 | $-CH_3$ | 1,43 (d,6H); 1,6 (m,2H); 2,48 (m,2H); 3,38 (sept.,1H); 3,47 (s,3H); 3,72 (s, 3H); 4,21 (m,1H); 4,38 (m,1H); 5.22 (dd,1H); 6,56 (d,1H); 6,70 - 7,20 (m,8H). |

Fortsetzung Tabelle 1

| Bsp.-Nr. | $R^1$ | $R^2$ | $R^3$ | $R^4$ | $^1H$-NMR [δ ppm, $CDCl_3$, 300 MHz] |
|---|---|---|---|---|---|
| 28 | $-CH(CH_3)_2$ | 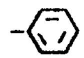 | 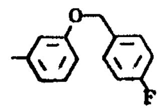 | $-CH_3$ | 1,43 (d,6H); 1,6 (m,2H); 2,46 (m,2H); 3,38 (sept.,1H); 3,48 (s,3H); 3,72 (s, 3H); 4,18 (m,1H); 4,35 (m,1H); 4,78 (s, 2H); 5,23 (dd,1H); 6,57 (d,1H); 6,70 - 7,40 (m,13H). |
| 29 | $-CH(CH_3)_2$ | 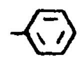 | 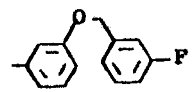 | $-CH_3$ | 1,43 (d,6H); 1,6 (m,2H); 2,47 (m,2H); 3,37 (sept.,1H); 3,48 (s,3H); 3,72 (s,3H); 4,19 (m,1H); 4,37 (m,1H); 4,85 (s,2H); 5,22 (dd,1H); 6,50 - 7,30 (m,14H). |
| 30 | $-CH(CH_3)_2$ | 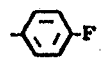 | 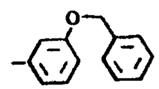 | $-CH_3$ | 1,43 (d,6H); 1,6 (m,2H); 2,47 (m,2H); 3,35 (sept.,1H); 3,44 (s,3H); 3,72 (s, 3H); 4,22 (m,1H); 4,38 (m,1H); 5,00 (s,2H); 5,22 (dd,1H); 6,57 (d,1H); 6,60 - 7,40 (m,13H). |
| 31 | $-CH(CH_3)_2$ | 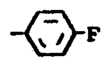 | 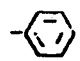 | $-CH_2CH_3$ | 1,12 (t,3H); 1,43 (d,6H); 1,6 (m,2H); 2,47 (m,2H); 3,28 (sept.,1H); 3,72 (s, 3H); 3,83 (q,2H); 4,18 (m,1H); 4,36 (m, 1H); 5,13 (dd,1H); 6,58 (d,1H); 6,70 - 7,30 (m,9H). |
| 32 | $-CH(CH_3)_2$ | 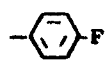 | 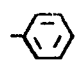 | $-CH(CH_3)_2$ | 1,45 (m,12H); 1,6 (m,2H); 2,47 (m,2H); 3,42 (sept.,1H); 3,70 (s,3H); 4,17 (m, 1H); 4,33 (m,1H); 4,41 (m,1H); 5,06 (dd, 1H); 6,70 (d,1H); 6,70 - 7,30 (m,9H). |
| 33 | $-CH(CH_3)_2$ | 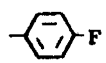 | 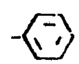 | -H | 1,45 (d,6H); 1,6 (m,2H); 2,47 (m,2H); 3,30 (sept.,1H); 3,75 (s,1H); 4,22 (m, 1H); 4,37 (m,1H); 5,37 (dd,1H); 6,42 (d, 1H); 6,90 - 7,30 (m,9H); 8,0 (bs,1H). |

Beispiel 34

3-(4-Fluorphenyl)-5-isopropyl-1,2-diphenyl-pyrrol

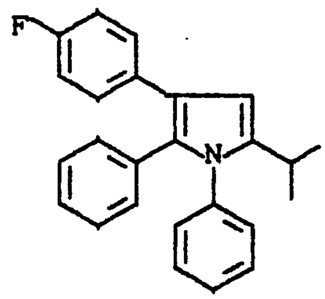

10,1 g (34 mmol) 2-(4-Fluorphenyl)-5-methyl-1-phenyl-hexan-1,4-dion (Beispiel 2) und 9,3 mmol Anilin (102 mmol) werden in 150 im Toluol unter Zugabe von 500 mg p-Toluolsulfonsäure 24 h am Wasserabscheider unter Rückfluß erhitzt. Nach Abkühlen und Verdünnen mit Essigsäureethylester wird mit 1 N-Salzsäure und anschließend mit Natriumhydrogencarbonat-Lösung gewaschen, über Magnesiumsulfat getrocknet und im Vakuum eingeengt. Abschließend wird über Kieselgel chromatographiert.

Ausbeute: 4,4 g (37% der Theorie)

$^1$H-NMR ($CDCl_3$): $\delta$ = 1,17 (d, 6H); 2,70 (sept., 1H); 6,28 (s, 1H); 6,80 - 7,30 (m, 14H).

In Analogie zu Beispiel 6 und den in den Beispielen 1, 2, 33 4, 5 beschriebenen Reaktionen wurden die in Tabelle 2 aufgeführten Verbindungen erhalten.

Tabelle 2

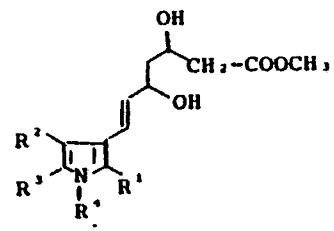

| Bsp.-Nr. | $R^1$ | $R^2$ | $R^3$ | $R^4$ | $^1$H-NMR [δ ppm, $CDCl_3$, 300 MHz] |
|---|---|---|---|---|---|
| 35 | $-CH(CH_3)_2$ | 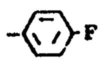 | 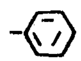 | 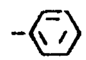 | 1,27 (d,6H); 1,47 (m,1H); 1,60 (m,1H); 2,47 (m,2H); 2,97 (sept., 1H); 3,72 (s, 3H); 4,20 (m,1H); 4,38 (m,1H); 5,21 (dd, (1H); 5,75 (d,1H); 6,80 - 7,40 (m,14H). |
| 36 | $-CH(CH_3)_2$ | 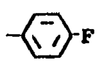 | 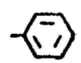 | 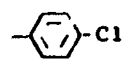 | 1,27 (d,6H); 1,55 (m,2H); 2,48 (m,2H); 2,95 (sept.,1H); 3,72 (s,3H); 4,19 (m, 1H); 4,38 (m,1H); 5,20 (dd,1H); 6,73 (d, 1H); 6,80 - 7,30 (m,13H). |
| 37 | $-CH(CH_3)_2$ | 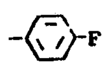 | 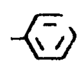 | 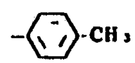 | 1,27 (m,6H); 1,60 (m,2H); 2,33 (s,3H); 2,47 (m,2H); 3,02 (sept.,1H); 3,72 (s, 3H); 4,20 (m,1H); 4,41 (m,1H); 5,21 (dd, 1H); 6,70 - 7,30 (m,14H). |
| 38 | $-CH(CH_3)_2$ | 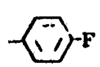 | 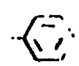 | 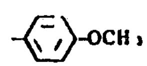 | 1,25 (d,6H); 1,55 (m,2H); 2,47 (m,2H); 2,95 (m,1H); 3,72 (s,3H); 3,78 (s,3H); 4,21 (m,1H); 4,38 (m,1H); 5,20 (dd,1H); 6,70 - 7,30 (m,14H). |

Fortsetzung Tabelle 2

| Bsp.-Nr. | $R^1$ | $R^2$ | $R^3$ | $R^4$ | $^1H$-NMR [δ ppm, $CDCl_3$, 300 MHz] |
|---|---|---|---|---|---|
| 39 | $-CH(CH_3)_2$ | 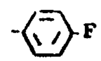 | 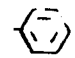 | 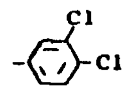 | 1,29 (m,6H); 1,45 (m,1H); 2,47 (m,2H); 2,92 (sept.,1H); 3,72 (s,3H); 4,18 (m, 1H); 4,38 (m,1H); 5,20 (dd,1H);6,69 (d,1H); 6,80 - 7,30 (m,12H). |
| 40 | $-CH(CH_3)_2$ | 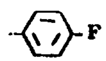 | 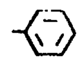 | 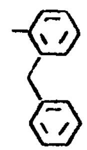 | 1,25 (m,6H); 1,45 (m,1H); 1,60 (m,1H); 2,47 (m,2H); 2,76 (sept.,1H); 3,42 (m, 2H); 3,72 (s,3H); 4,18 (m,1H); 4,38 (m,1H); 5,19 (m,1H); 6,70 - 7,45 (m,19H). |
| 41 | $-CH(CH_3)_2$ | 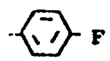 | 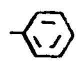 | $-CH_2-$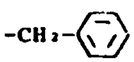 | 1,27 (d,6H); 1,55 (m,2H); 2,47 (m,2H); 3,02 (sept.,1H); 3,72 (s,3H); 4,20 (m, 1H); 4,37 (m,1H); 5,05 (s,2H); 5,16 (dd, 1H); 6,71 (d,1H); 6,80 - 7,30 (m,14H). |
| 42 | $-CH(CH_3)_2$ | 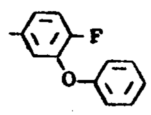 | 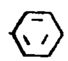 | 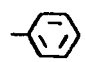 | 1,23 (d,6H); 1,55 (m,2H); 2,47 (m,2H); 2,93 (sept.,1H); 3,72 (s,3H); 4,24 (m. 1H); 4,41 (m,1H); 5,30 (dd,1H); 6,70-7,40 (m,19H). |

Beispiel 43

2-(4-Fluorphenyl)-1,5-dimethyl-hexan-1,4-dion

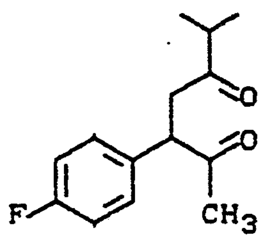

Zu einer Lösung von 19,2 g (0,1 mol) 1-(4-Fluorphenyl)-4-methyl-pent-1-en-3-on und 8,6 ml (0,12 mol) Nitroethan in 100 ml Acetonitril p.a. tropft man bei 0°C eine Lösung von 7,9 g 0,05 mol) 1,5-Diazabicyclo-(5,4,0)undec-5-en in 50 ml Acetonitril p.a. Anschließend wird 3 h bei Raumtemperatur gerührt und 100 ml 0,5 N-Salzsäure werden zugegeben. Nach Extraktion mit Methylenchlorid wird die oganische Phase mit Natriumsulfat getrocknet und eingeengt. Der ölige Rückstand wird in 100 ml Ethanol gelöst und bei Raumtemperatur mit einer Lösung von 4,8 g (0,12 mol) Natriumhydroxid in 60 ml Wasser versetzt. Diese Reaktionslösung wird dann unter Eiskühlung zu einer Lösung von 13,3 ml (0,25 mol) konzentrierter Schwefelsäure in 100 ml Wasser getropft. Nach 0,5 h Rühren bei Raumtemperatur wird mit Wasser verdünnt und mehrmals mit Ether extrahiert. Die organische Phase wird je zweimal mit 0,5 N-Salzsäure und gesättigter Natriumhydrogencarbonatlösung gewaschen, über Magnesiumsulfat getrocknet, im Vakuum eingeengt und über Kieselgel chromatographiert.
Ausbeute: 14,7 g (62,3% der Theorie)
$^1$H-NMR ($CDCl_3$): $\delta$ = 1,07 (d, 3H); 1,12 (d, 3H); 2,13 (s, 3H); 2,60 (m, 2H); 3,42 (dd, 1H); 4,24 (dd, 1H); 7,00 - 7,30 (m, 4H).

In Analogie zu Beispiel 6 und den in den Beispielen 1, 43, 3 bzw. 34, 4 und 5 beschriebenen Reaktionen wurden die in Tabelle 3 aufgeführten Verbindungen erhalten.

Tabelle 3

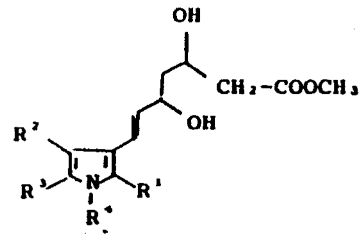

| Bsp.-Nr. | $R^1$ | $R^2$ | $R^3$ | $R^4$ | $^1$H-NMR [δ ppm, $CDCl_3$, 300 MHz] |
|---|---|---|---|---|---|
| 44 | $-CH(CH_3)_2$ | 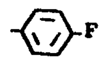 | $-CH_3$ | $-CH_3$ | 1,37 (d,6H); 1,55 (m,2H); 2,09 (s,3H); 2,47 (m,2H); 3,30 (m,1H); 3,52 (s,3H); 3,72 (s,3H); 4,20 (m,1H); 4,33 (m,1H); 5,12 (dd,1H); 6,53 (d,1H); 6,90 - 7,20 (m,4H). |
| 45 | $-CH(CH_3)_2$ | 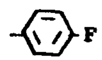 | $-CH_3$ | 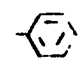 | 1,20 (d,6H); 1,55 (m,2H); 1,82 (s,3H); 2,47 (m,2H); 2,87 (sept.,1H); 3,72 (s, 3H); 4,18 (m,1H); 4,37 (m,1H); 5,18 (dd, 1H); 6,68 (d,1H); 6,90 - 7,50 (m,9H). |
| 46 | $-CH(CH_3)_2$ | 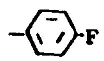 | $-CH_3$ | 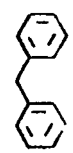 | 1,18 (d,3H); 1,28 (d,3H); 1,50 - 1,60 (m,5H); 2,47 (m,2H); 2,69 (sept.,1H); 3,63 (m,2H); 3,72 (s,3H); 4,19 (m,1H); 4,37 (m,1H); 5,17 (dd,1H); 6,71 (d,1H); 7,00 - 7,50 (m,13H). |
| 47 | $-CH(CH_3)_2$ | 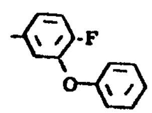 | $-CH_3$ | $-CH_3$ | 1,32 (d,6H); 1,55 (m,2H); 2,08 (s,3H); 2,47 (m,2H); 3,28 (sept.,1H); 3,48 (s, 3H); 3,72 (s,3H); 4,20 (m,1H); 4,33 (m, 1H); 5,19 (dd,1H); 6,50 (d,1H); 6,90 - 7,40 (m,8H). |
| 48 | $-CH(CH_3)_2$ | 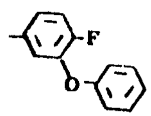 | $-CH_3$ | 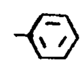 | 1,18 (d,6H); 1,55 (m,2H); 1,80 (s,3H); 2,47 (m,2H); 2,85 (sept.,1H); 3,72 (s, 3H); 4,23 (m,1H); 4,38 (m,1H); 5,26 (dd, 1H); 6,63 (d,1H); 7,00 - 7,50 (m,13H). |

Beispiel 49

Methyl-erythro-(E)-7-[3-(4-fluorphenyl)-5-isopropyl-1-methyl-2-phenyl-pyrrol-4-yl]-3,5-dihydroxy-heptanoat

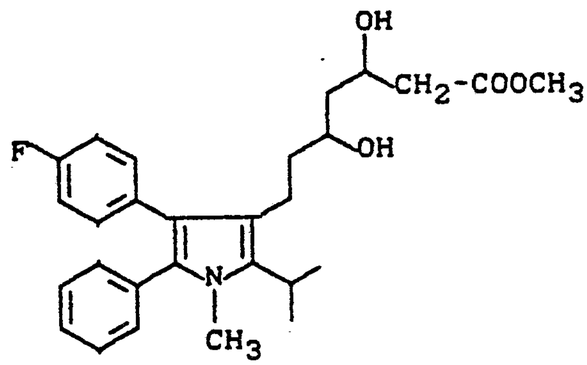

100 mg der Verbindung aus Beispiel 6 werden in 20 ml Methanol und 10 µl Triethylamin gelöst und nach Zugabe von 15 mg 10%iger Palladium auf Tierkohle 3,5 h bei 2,5 bar hydriert. Der Katalysator wird abfiltriert und die Lösung wird im Vakuum eingeengt.
Ausbeute: 91 mg(90% der Theorie)
$^1$H-NMR ($CDCl_3$): δ = 1,45 (m, 10H); 2,48 (m, 2H); 2,60 (m, 2H); 2,97 (m, 1H); 3,28 (m, 1H); 3,49 (s, 3H); 3,70 (s,3H); 4,12 (m, 1H); 6,80 - 7,30 (m, 9H).

Anwendungsbeispiel

Beispiel 50

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat (Mischung aus $K_2HPO_4$ und $KH_2PO_4$ mit pH 7,2), 0,03 m Ethylendiamintetraessigsäure, 0,002 m Dithiothreit (SPE)-Puffer (Saccharose-Phosphat-Ethylendiamintetraessigsäure-Puffer) pH 7,2, homogenisiert. Anschließend wurde 15 Minuten zentrifugiert und das Sediment verworfen. Der Überstand wurde 75 Minuten bei 100 000 g sedimentiert. Das Pellet wird in 1/4 Volumen SPE-Puffer aufgenommen, nochmals homogenisiert und anschließend erneut 60 Minuten zentrifugiert. Das Pellet wird mit der 5-fachen Menge ihres Volumens SPE-Puffer aufgenommen, homogenisiert und bei -78°C eingefroren und gelagert (Enzymlösung).

Zur Testung werden die Testverbindungen (bzw. Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP (ß-Nicotinamid-adenin-dinucleolid-phosphat), 1 Einheit Glucose-6-Phosphatdehydrogenase 35 µMol $K_xH_y$-Phosphat pH 7,2, 20 µl Enzympräparation und 56 nMol 3-Hydroxy-3-methyl-glutaryl Coenzym A (Glutaryl-3-$^{14}$C) 100 000 dpm.

Man inkubierte 60 Minuten bei 37°C und stoppte die Reaktion durch Zusatz von 300 µl 0,24 m HCl ab. Nach einer Nachinkubation von 60 Minuten bei 37°C wurde der Ansatz zentrifugiert und 600 µl des

Überstandes auf eine 0,7 x 4 cm mit 5-Chlorid Anionenaustauscher mit einer Korngröße von 100 bis 200 mesh gefüllte Säule aufgetragen. Es wurde mit 2 ml dest. Wasser nachgewaschen und Durchlauf plus Waschwasser mit 3 ml einer Scintillationsflüssigkeit versetzt und im Scintillationszähler gezählt. $IC_{50}$-Werte wurden durch Auftrag der prozentualen Hmmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 100 gesetzt und mit dem simultan bestimmten $IC_{50}$-Wert der Testverbindung verglichen.

Die erfindungsgemäßen Wirkstoffe haben eine höhere inhibitorische Potenz als Mevinolin.

Beispiel 51

Die subchronische Wirkung der erfindungsgemäßen Verbindungen auf die Blutcholesterinwerte von Hunden wurde in mehrwöchigen Fütterungsexperimenten geprüft. Dazu wurde die zu untersuchende Substanz über einen mehrwöchigen Zeitraum einmal täglich in einer Kapsel gesunden Beagle Hunden zusammen mit dem Futter p.o. gegeben. Dem Futter war außerdem während der gesamtem Versuchsperiode, d.h. vor, während und nach der Applikationsperiode der zu untersuchenden Substanz, Colestyramin (4 g/100 g Futter) als Gallensäuresequestrant beigemischt. Zweimal wöchentlich wurde den Hunden venöses Blut entnommen und das Serumcholesterin enzymatisch bestimmt. Die Serumchlolesterinwerte während der Applikationsperiode wurden mit den Serumcholesterinwerten vor der Applikationsperiode (Kontrolle) verglichen.

So ergab sich z.B. für den Wirkstoff nach Beispiel 6 nach 2 wöchiger Applikation von täglich 2 mg/kg p.o. eine Senkung des Serumcholesterins um 27 %.

**Ansprüche**

1. Substituierte Pyrrole der Formel

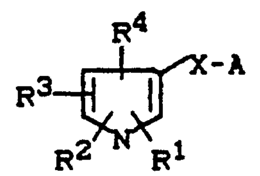

(I)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkythio oder Aralkylsulfonyl, wobei die Heteroaryl-und Arylreste der letztgenannten Substituenten bis zu 3-fach gleich oder ver schieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
wobei $R^3$ auch für Alkyl stehen kann,
$R^4$ -für Wasserstoff oder
-für Acyl, Alkylsulfonyl oder Arylsulfonyl steht, wobei der Arylrest durch Alkyl oder Halogen substituiert sein kann, oder
-für Aminocarbonyl, Alkylaminocarbonyl, Dialkylaminocarbonyl, Arylaminocarbonyl oder Alkoxycarbonyl steht, oder
-für Cycloalkyl steht, oder
-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder
-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
X - für Gruppe der Formel $-CH_2-CH_2-$oder $-CH=CH-$steht,
und
A - für eine Gruppe der Formel

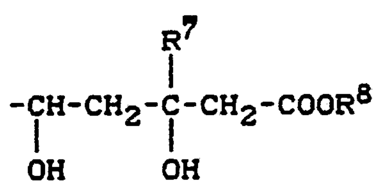

oder

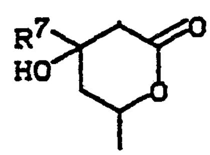

steht,
worin
$R^7$ -Wasserstoff oder Alkyl bedeutet
und
$R^8$ -Wasserstoff,
- Alkyl, Aryl oder Aralkyl oder
-ein Kation bedeutet.

2. Substituierte Pyrrole nach Anspruch 1
worin
$R^1$ - für Cyclopropyl, Cyclopentyl oder Cyclohexyl steht, oder
-für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ gleich oder verschieden sind und Niederalkyl, Phenyl, Benzyl, Acetyl, Benzoyl, Phenylsulfonyl

oder Niederalkylsulfonyl bedeuten,
oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,
$R^2$ und $R^3$gleich oder verschieden sind und
-für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
-für Phenyl oder Naphthyl stehen, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ die oben angegebene Bedeutung haben,
wobei
$R^4$ -für Wasserstoff oder
-für Benzoyl oder Niederalkylcarbonyl steht, oder
-für Niederalkylsulfonyl,Phenylsulfonyl oder Tolysulfonyl steht, oder
-für Aminocarbonyl, Niederalkylaminocarbonyl, Diniederalkylaminocarbonyl, Phenylaminocarbonyl oder Niederalkoxycarbonyl steht, oder
-für Cyclopropyl, Cyclopentyl oder Cyclohexyl steht oder
-für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ gleich oder verschieden sind und Niederalkyl, Phenyl, Benzyl, Acetyl, Benzoyl, Phenylsulfonyl oder Niederalkylsulfonyl bedeuten,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Nieder alkoxy, Trifluormethyl, oder Trifluormethoxy substituiert sein können,
-für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
-für Phenyl oder Naphthyl stehen, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ die oben angegebene Bedeutung haben
X - für eine Gruppe der Formel $-CH_2-CH_2-$oder $-CH=CH-$steht,
A - für eine Gruppe der Formel

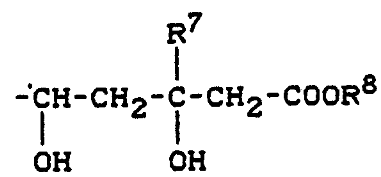

oder

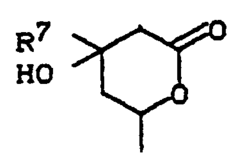

steht,
worin
$R^7$ -Wasserstoff oder Niederalkyl bedeutet, und
$R^8$ -Wasserstoff,
-Alkyl ($C_1$ bis $C_4$), Aryl ($C_6$ bis $C_{12}$), Aralkyl ($C_7$ bis $C_{10}$)
oder
-ein physiologisch verträgliches Kation bedeutet.

3. Substituierte Pyrrole nach den Ansprüchen 1 und 2, worin
$R^1$ - für Cyclopropyl, Cylcopentyl oder Cyclohexyl steht, oder
-für Methyl, Ethyl, Propyl, Isopropyl, Butyl, sec.-Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
$R^2$ und $R^3$ gleich oder verschieden sind und
-für Pyridyl, Pyrimidyl, Chinolyl oder Isochinolyl stehen, die durch Fluor, Chlor, Methyl, Methoxy oder Trifluormethyl substituiert sein können, oder
-für Phenyl stehen, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
$R^4$ -für Wasserstoff, Cyclopropyl, Cyclopentyl oder Cyclohexyl steht, oder
-für Methyl, Ethyl, Propyl, Isopropyl, Butyl, Isobutyl, tert.Butyl, Pentyl, Isopentyl, Hexyl oder Isohexyl steht, die substituiert sein können durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.-Butoxycarbonyl, Benzoyl, Acetyl, Ethylcarbonyl, oder durch eine Gruppe $-NR^5R^6$,
wobei
$R^5$ und $R^6$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolinyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl-und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
-für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl steht, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
-für Phenyl steht, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.-Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^5R^6$ substituiert sein kann,
wobei
$R^5$ und $R^6$ die oben angegebene Bedeutung haben,
oder

$R^4$ -für Benzoyl, Acetyl, Ethylcarbonyl, Propylcarbonyl, Isopropylcarbonyl, Methylsulfonyl, Ethylsulfonyl, Propylsulfonyl, Isopropylsulfonyl, Butylsulfonyl, Isobutylsulfonyl, tert.Butylsulfonyl, Phenylsulfonyl oder Tolylsulfonyl steht,

-für Aminocarbonyl, Methyl-, Ethyl-, Propyl-, Isopropyl-, Butyl-, Isobutyl-oder tert.Butylaminocarbonyl, Dimethyl-, Diethyl-, Dipropyl-, Diisopropyl-, Dibutyl-oder Diisobutylaminocarbonyl, Phenylaminocarbonyl,Methoxycarbonyl, Ethoxycarbonyl, Propoxycarbonyl, Isopropoxycarbonyl, Butoxycarbonyl, Isobutoxycarbonyl oder tert.Butoxycarbonyl steht,

X - für eine Gruppe der Formel $-CH_2-CH_2-$oder $-CH=CH-$steht,

A - für eine Gruppe der Formel

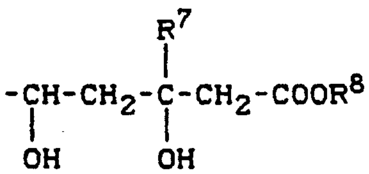

oder

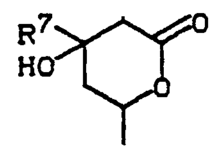

steht,

worin

$R^7$ -Wasserstoff, Methyl, Ethyl, Propyl, Isopropyl, Butyl, Isobutyl oder tert.Butyl bedeutet,

und

$R^8$ -Wasserstoff, Methyl, Ethyl, Propyl, Isopropyl, Butyl, Isobutyl, tert.Butyl oder Benzyl bedeutet, oder ein Natrium-, Kalium-, Calcium-, oder Magnesium-oder Ammoniumion bedeutet.

4. Substituierte Pyrrole nach den Ansprüchen 1 bis 3, worin

$R^1$ - für Cyclopropyl, Methyl, Ethyl, Propyl, Isopropyl, Butyl, Isobutyl oder tert.Butyl steht, das durch Fluor, Chlor, Methoxy, Phenyl oder Phenoxy substituiert sein kann,

$R^2$ und $R^3$ - gleich oder verschieden sind und

-für Phenyl stehen, die bis zu 2-fach gleich oder verschieden durch Methyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Brom, Cyano, Trifluormethyl oder Trifluormethoxy substituiert sein können,

$R^4$ -für Wasserstoff, Cyclopropyl, Cyclopentyl oder Cyclohexyl·steht, oder

-für Methyl, Ethyl, Propyl, Isopropyl, Butyl, Isobutyl, tert.Butyl, Pentyl, Isopentyl, Hexyl oder Isohexyl steht, das substituiert sein kann durch Fluor, Chlor, Methoxy, Ethoxy, Propoxy, Isopropoxy, Methylthio, Ethylthio, Methylsulfonyl, Ethylsulfonyl, Trifluormethyl, Trifluormethoxy, Methoxycarbonyl, Ethoxycarbonyl, Propoxycarbonyl, Isopropoxycarbonyl oder durch eine Gruppe der Formel $-NR^5R^6$,

wobei

$R^5$ und $R^6$ -gleich oder verschieden sind und

-Wasserstoff, Methyl, Ethyl, Propyl, Isopropyl, Phenyl oder Benzyl bedeuten,

oder durch gegebenenfalls durch Fluor, Chlor, Methyl, Methoxy, Trifluormethyl oder Trifluormethoxy substituiertes Pyridyl, Pyrimidyl, Chi nolyl, Thienyl, Furyl, Phenyl, Phenoxy, Phenylsulfonyl oder Benzyloxy oder

-für gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Benzthiazolyl oder Benzimidazolyl steht, oder

-für Phenyl steht, das bis zu 2-fach gleich oder verschieden durch Methyl, Ethyl, Propyl, Isopropyl, Butyl, Isobutyl, tert.Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel $-NR^5R^6$ substituiert sein kann,

wobei

$R^5$ und $R^6$ die oben angegebene Bedeutung haben,

oder

$R^4$ -für Acetyl, Methylsulfonyl, Ethylsulfonyl, Phenylsulfonyl, Tolylsulfonyl, Aminocarbonyl, Methylaminocarbonyl, Dimethylaminocarbonyl, Phenylaminocarbonyl, Methoxycarbonyl, Ethoxycarbonyl, Propoxycarbonyl, Isopropoxycarbonyl, Butoxycarbonyl, Isobutoxycarbonyl oder tert.Butoxycarbonyl steht,

X - für eine Gruppe der Formel

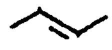 (E-konfiguriert)

steht und A - für eine Gruppe der Formel

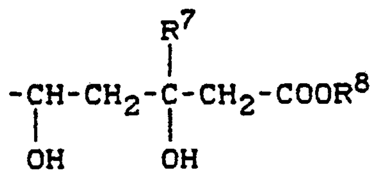 oder 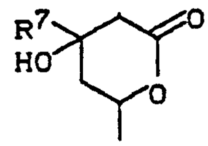

steht, worin

$R^7$ -Wasserstoff bedeutet

und

$R^8$ -Wasserstoff, Methyl oder Ethyl bedeutet oder ein Natrium-oder Kaliumkation bedeutet.

5. Substituierte Pyrrole der Formel

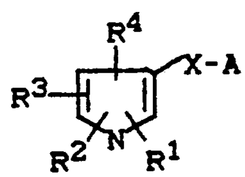

(I)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroarylund Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Tri fluormethylthio, Alkoxycarbonyl oder durch eine Gruppe der Formel $-NR^5R^6$ substituiert sein kann,

worin

$R^5$ und $R^6$die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

X - für eine Gruppe der Formel $-CH_2CH_2-$oder $-CH=CH-$steht,

und

A - für eine Gruppe der Formel

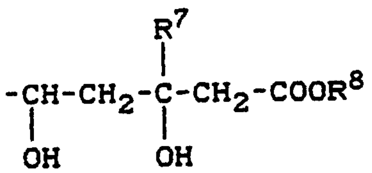

oder

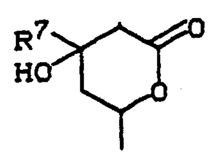

steht,

worin

$R^7$ -Wasserstoff oder Alkyl bedeutet

und

$R^8$ -Wasserstoff,

-Alkyl, Aryl oder Aralkyl oder

-ein Kation bedeutet,

zur therapeutischen Verwendung.

6. Verfahren zur Herstellung von substituierten Pyrrolen der Formel

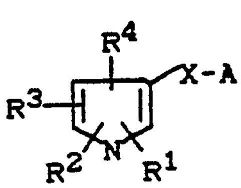

(I)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl, oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Tri fluormethylthio, Alkoxycarbonyl oder durch eine Gruppe der Formel $-NR^5R^6$ substituiert sein kann,

worin

$R^5$ und $R^6$ die oben angegebene Bedeutung haben, oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben

X - für eine Gruppe der Formel $-CH_2-CH_2$ oder $-CH=CH-$steht,

und

A - für eine Gruppe der Formel

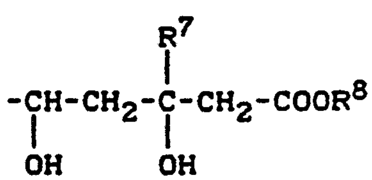

oder

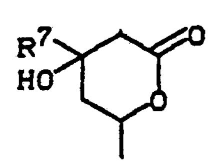

steht,

worin

$R^7$ -Wasserstoff oder Alkyl bedeutet

und
$R^8$ -Wasserstoff,
- Alkyl, Aryl oder Aralkyl oder
-ein Kation bedeutet,
dadurch gekennzeichnet, daß man
Ketone der allgemeinen Formel

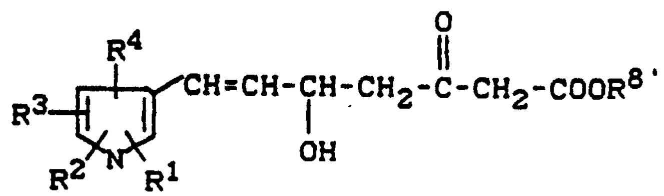

in welcher
$R^1$, $R^2$, $R^3$ und $R^4$ die oben angegebene Bedeutung haben,
und
$R^{8'}$ -für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen (X = $-CH_2-CH_2-$) die Ethenverbindungen (X= -CH=CH-) nach üblichen Methoden hydriert,
und gegebenenfalls Isomeren trennt.

7. Ketone der Formel

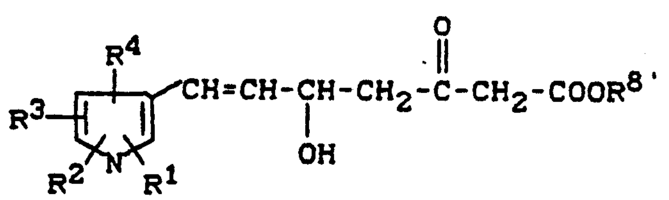

in welcher
$R^1$ - für Cycloalkyl steht, oder
-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ und $R^3$ - gleich oder verschieden sind und
-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder
-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

-für Heteroaryl stehen, das bis zu 3-fach gleich oder verschieden durch Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio,Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

X - für eine Gruppe der Formel $-CH_2-CH_2$ oder $-CH=CH$-steht,

und

$R^{8'}$ -für Alkyl steht.

8. Verfahren zur Herstellung von Ketonen der Formel

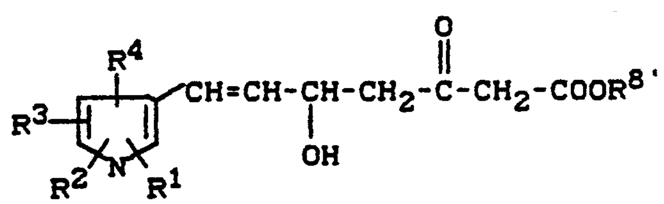

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,
oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
$R^4$ -für Wasserstoff oder

-für Acyl, Alkylsulfonyl oder Arylsulfonyl steht, wobei der Arylrest durch Alkyl oder Halogen sub stituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio der Aralkylsulfonyl, wobei die Heteroaryloder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

-für Heteroaryl stehen, das bis zu 3-fach gleich oder verschieden durch Alkyl, Alkoxy, Alkylthio, Alkylsulfonyl, Aryl, Aryloxy, Arylthio, Arylsul fonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$die oben angegebene Bedeutung haben,
oder

-für Aryl steht, das bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio,Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
X - für eine Gruppe der Formel $-CH_2-CH_2$ oder $-CH=CH$-steht,
und
$R^{8'}$ -für Alkyl steht,
dadurch gekennzeichnet, daß man
Aldehyde der allgemeinen Formel (IX)

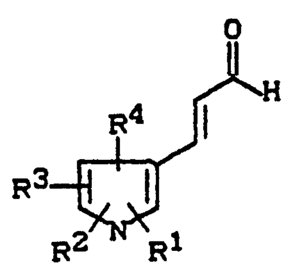

(IX)

in welcher
$R^1$, $R^2$, $R^3$ und $R^4$ die oben angegebene Bedeutung haben,
in inerten Lösemitteln mit Acetessigester der allgemeinen Formel (X)

$$H_3C-\overset{O}{\overset{\|}{C}}-CH_2-COOR^{8'} \quad (X)$$

in welcher
$R^{8'}$ die oben angegebene Bedeutung hat,

in Anwesenheit von Basen umsetzt.

9. Aldehyde der Formel

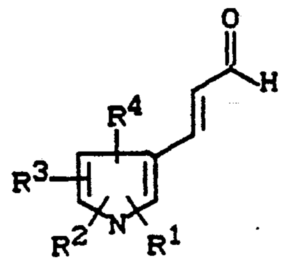

(IX)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben.

10. Verfahren zur Herstellung von Aldehyden der Formel

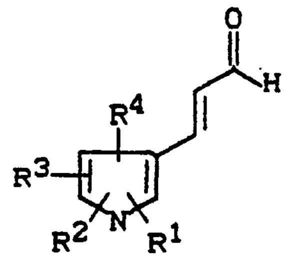

(IX)

in welcher
$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin

$R^5$ und $R^6$ die oben angegebene Bedeutung haben

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

-für Heteroaryl stehen, das bis zu 3-fach gleich oder verschieden durch Halogen, Alkyl, Alkoxy,

Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^5R^6$ substituiert sein kann,
worin
$R^5$ und $R^6$ die oben angegebene Bedeutung haben,
oder
-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
dadurch gekennzeichnet, daß man Pyrrole der allgemeinen Formel (XI)

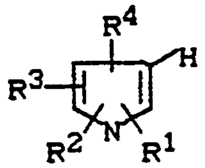

(XI)

in welcher
$R^1$, $R^2$, $R^3$ und $R^4$ die oben angegebene Bedeutung haben,
in inerten Lösemitteln in Anwesenheit von Hilfsstoffen mit N,N-Dimethylaminoacrolein der Formel (XII)

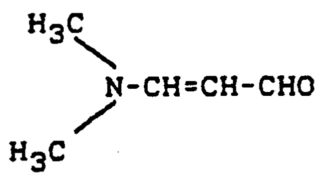

(XII)

umsetzt.

11. Arzneimittel, enthaltend substituierte Pyrrole der Formel

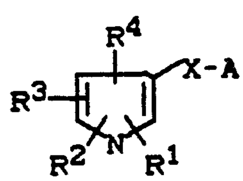

(I)

in welcher
$R^1$ - für Cycloalkyl steht, oder
-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ und $R^3$ - gleich oder verschieden sind und
-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Tri fluormethylthio, Alkoxycarbonyl oder durch eine Gruppe der Formel $-NR^5R^6$ substituiert sein kann,
worin
$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

X - für eine Gruppe der Formel $-CH_2CH_2-$oder $-CH=CH-$steht,

und

A - für eine Gruppe der Formel

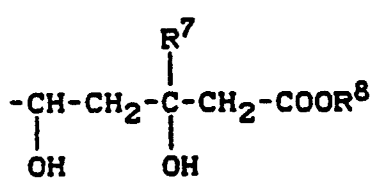

oder

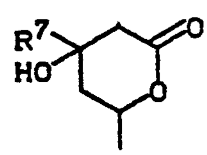

steht,

worin

$R^7$ -Wasserstoff oder Alkyl bedeutet

und

$R^8$ -Wasserstoff,

-Alkyl, Aryl oder Aralkyl oder

-ein Kation bedeutet.

12. Arzneimittel nach Anspruch 11, dadurch gekennzeichnet, daß sie 0,5 bis 98 Gew.-% der substituierten Pyrrole enthalten.

13. Verwendung von substituierten Pyrrolen der Formel

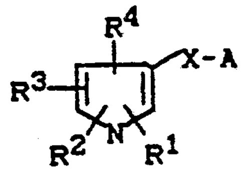

(I)

in welcher

$R^1$ - für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder veschieden durch Halogen, Cyano, Trifluormethyl, Trifluormethoxy, Alkyl, Alkoxy, Alkylthio oder Alkylsulfonyl substituiert sein können,

$R^2$ und $R^3$ - gleich oder verschieden sind und

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^4$ -für Wasserstoff oder

-für Acyl, Alkylsulfonyl oder Arylsulfonyl steht, wobei der Arylrest durch Alkyl oder Halogen substituiert sein kann, oder

-für Aminocarbonyl, Alkylaminocarbonyl, Dialkylaminocarbonyl, Arylaminocarbonyl oder Alkoxycarbonyl steht, oder

-für Cycloalkyl steht, oder

-für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ -gleich oder verschieden sind und Alkyl, Aryl, Aralkyl, Acyl, Alkylsulfonyl oder Arylsulfonyl bedeuten, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

-für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,
X - für eine Gruppe der Formel $-CH_2CH_2-$oder $-CH=CH-$steht,
und
A - für eine Gruppe der Formel

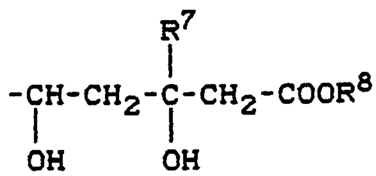

oder

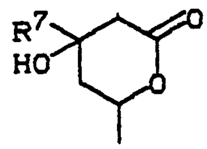

steht,
worin
$R^7$ -Wasserstoff oder Alkyl bedeutet
und
$R^8$ -Wasserstoff,
-Alkyl, Aryl, Aralkyl oder
-ein Kation bedeutet,
zur Herstellung von Arzneimitteln.

14. Verwendung nach Anspruch 13 zur Herstellung von Inhibitoren der 3-Hydroxy-3-methyl-glutaryl Coenzym A (HMG-CoA)-Reduktase.

Europäisches Patentamt

# EUROPÄISCHER RECHERCHENBERICHT

Nummer der Anmeldung: EP 88105456.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 009 326 (PFIZER CORPORATION)<br>* Patentansprüche 1,9 * | 1,11 | C 07 D 207/337<br>C 07 D 207/333<br>A 61 K 31/40 |
| A | AT - B - 269 866 (RÜTGERSWERKE AKTIENGESELLSCHAFT)<br>* Patentanspruch 1 * | 1 | |
| A | CH - A - 529 131 (MC NEIL LABORATIORIES INCORPORATED)<br>* Patentanspruch I * | 1 | |
| A | DE - C - 564 126 (I.G. FARBEN-INDUSTRIE AKT.-GES.)<br>* Seite 1 * | 1 | |

RECHERCHIERTE SACHGEBIETE (Int. Cl.4)

C 07 D 207/00
A 61 K 31/00
C 07 D 405/00
C 07 D 403/00
C 07 D 401/00

Der vorliegende Recherchenbericht wurde für alle Patentansprüche erstellt.

| Recherchenort | Abschlußdatum der Recherche | Prüfer |
|---|---|---|
| WIEN | 30-06-1988 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument